# EUROPEAN PATENT SPECIFICATION

(11) **EP 2 117 429 B1**
(45) Date of publication and mention of the grant of the patent: **10.06.2020**
(21) Application number: 08728744.7
(22) Date of filing: 31.01.2008
(51) Int. Cl.: A61B 5/08, A61B 5/087, A61B 5/097, A61B 5/083

(54) **METABOLIC MEASUREMENT SYSTEM INCLUDING A MULTIPLE FUNCTION AIRWAY ADAPTER**
STOFFWECHSELMESSSYSTEM MIT EINEM MULTIFUNKTIONS-ATEMWEGSADAPTER
SYSTÈME DE MESURE MÉTABOLIQUE À ADAPTATEUR DE VOIE AÉRIENNE MULTIFONCTION

(30) Priority: 01.02.2007 US 701187
(43) Date of publication of application: 18.11.2009
(73) Proprietor: RIC Investments, LLC., Wilmington, DE 19801-1545 (US)
(72) Inventor: MACE, Leslie, E., Mercer Island, WA 98040 (US); ORR, Joseph, A., Park City, UT 84098 (US); RICH, David, R., Glastonbury, CT 06033 (US); JAFFE, Michael, B., Cheshire, CT 06410 (US); ALDERETE, Jason, Durham, CT 06422 (US)
(74) Representative: de Haan, Poul Erik
(86) International application number: PCT/US2008/052690
(87) International publication number: WO 2008/095120

(56) References cited:
- WO-A1-02/085207
- WO-A2-2006/102538
- WO-A2-2006/102538
- US-A1- 2002 029 003
- US-A1- 2006 069 326

## Description

### TECHNICAL FIELD

The present invention relates to metabolic measurement system that uses a multi-function airway adapter, which monitors the amounts of oxygen (O₂) in the respiration of an individual, as well as the respiratory flow of the amount of one or more of carbon dioxide (CO₂), nitrous oxide (N₂O), or an anesthetic agent other than nitrous oxide in the respiration of the individual. More specifically, the present invention relates to a metabolic measurement system that uses an integrated airway adapter, which is capable of monitoring, by luminescence quenching techniques, the fractions, or concentrations, of gases, such as O₂ in real time or breath-by-breath, as well as monitoring one or both of respiratory flow and, by infrared absorption techniques, the fractions, or concentrations, of gases such as CO₂, N₂O, and anesthetic agents.

### BACKGROUND OF THE INVENTION

### A. Respiratory Gas Monitoring

Various types of sensors that are configured to communicate with the airway of a patient to monitor substances such as gases or vapors in the respiration of the patient are known in the art. Molecular oxygen, carbon dioxide, and anesthetic agents, including nitrous oxide, are among the types of substances that may be detected with known sensors.

Typically, side-stream gas sensors are used during surgical procedures to indicate the condition of a patient to an anesthesiologist. Respiratory gas sensors may also be used in a variety of other medical procedures, such as heart stress tests with an individual on a treadmill, in other tests for monitoring the physical condition of an individual, and the like. Side-stream sampling requires the use of small bore sampling lines to draw gas from the breathing circuit for remote analysis. The problems associated with side-stream gas sampling are well known and include the following:
a) impeding of the sample line by the presence of water and patient secretions;
b) introduction of variable delay which creates synchronization difficulties when combining flow and gas concentration measures;
c) loss of signal fidelity due to low pass filtering; and
h) handling of exhaust, which may contain anesthetic agents, blood, secretions, etc.

The use of mainstream sensors to monitor respiratory and anesthetic gases has the potential to solve the problems associated with side-stream sensors, especially when combining gas and flow and/or pressure signals.

### B. Infrared Absorption

Infrared absorption has long been employed to detect and monitor gases, such as CO₂, N₂O, and other anesthetic agents, in the respiration of a patient. In infrared absorption (IR) techniques, infrared light of one or more wavelengths and of known intensity is directed into a stream of respiratory gases. The wavelength or wavelengths of such radiation are selected based on the gas or gases being analyzed, each of which absorbs one or more specific wavelengths of radiation. The intensity of the radiation which passes through the stream of respiratory gases, which radiation is typically referred to as "attenuated radiation", is measured and compared with the known intensity of the radiation emitted into the stream. This comparison of intensities provides information about the amount of radiation of each wavelength that is absorbed by each analyzed gas, which, in turn, provides information about the amount (i.e., the concentration or fraction) of that gas in the patient's respiration.

U.S. Patents 4,859,858 (hereinafter "the '858 Patent") and 4,859,859 (hereinafter "the '859 Patent"), both of which issued to Knodle et al. on August 22, 1989, and U.S. Patent 5,153,436 (hereinafter "the '436 Patent"), issued to Apperson et al. on October 6, 1992, each disclose apparatus that include infrared absorption type sensors for measuring the amount of one or more specific gases in the respiration of a patient.

Typically, infrared gas sensors, such as those disclosed in the'858, '859, and '436 Patents, include a source from which infrared radiation is emitted. The emitted infrared radiation is focused into a beam by a mirror. The beam is transmitted through a sample of the gases being analyzed. After passing through the gases, the infrared radiation beam passes through a filter. The filter reflects all of the radiation except for the radiation in a narrow band which corresponds to a frequency absorbed by the gas of interest. This narrow band of radiation is transmitted to a detector, which produces an electrical output signal proportional in magnitude to the magnitude of the intensity of the infrared radiation impinging upon the detector. As the intensity of the radiation that passes through the filter is attenuated to an extent that is proportional to the concentration of a gas of interest, the strength of the signal generated by the detector is inversely proportional to the concentration of the gas of interest.

Infrared (IR) type gas sensors that are configured to substantially simultaneously measure the amounts of more than one type of gas in the respiration of a patient are also known. One such sensor, disclosed in U.S. Patent 5,296,706 (hereinafter "the '706 Patent), issued to Braig et al. on March 22, 1994, includes a plurality of discrete channels for facilitating the independent detection of six or more different anesthetic agents. The article, Burte, E.P. et al., "Microsystems for measurement and dosage of volatile anesthetics and respirative gases in anesthetic equipment", MEMS 98 Proceedings., The Eleventh Annual International Workshop on Micro Electro Mechanical Systems, Pages 510-514 (1998) (hereinafter "the Burte Article"), discloses, among other things, a mainstream, multichannel sensor apparatus that is configured to simultaneously measure the amounts of a combination of anesthetic gases in the respiration of a patient.

Infrared type gas sensors typically employ a cuvette to sample the respiration of a patient via a nasal cannula or an endotracheal tube and a mechanical ventilator. The cuvette channels respiratory gases to a specific flow path and provides an optical path between an infrared radiation emitter and an infrared radiation detector, both of which can be detachably coupled to the cuvette.

A typical cuvette is molded from a polymer or other appropriate material and has a passage defining the flow path for the gases being monitored. The optical path crosses the flow path of the gases through windows in the sidewalls of the cuvette aligned along opposite sides of the flow passage, allowing the beam of infrared radiation to pass through the cuvette.

The windows are generally formed from sapphire because of sapphire's favorable optical properties. However, sapphire is a relatively expensive material. Consequently, these cuvettes are almost invariably cleaned, sterilized, and reused. The cleaning and sterilization of a cuvette is time consuming and inconvenient; and the reuse of a cuvette may pose a significant risk of contamination, especially if the cuvette was previously used in monitoring a patient suffering from a contagious and/or infectious disease.

Efforts have been made to reduce the cost of cuvettes by replacing the sapphire windows with windows fabricated from a variety of polymers. One of the major problems encountered in replacing sapphire cuvette windows with polymer windows is establishing and maintaining a precise optical path length through the sample being analyzed. This is attributable to such factors as a lack of dimensional stability in the polymeric material, the inability to eliminate wrinkles in the windows, and the lack of a system for retaining the windows at precise locations along the optical path.

Cuvette windows that are formed from polymers, including polypropylene, may limit the types of substances flowing through an airway adapter that may be monitored or measured by use of infrared techniques. This is because polymers typically include hydrocarbons, which may limit the transmissivity of polymers for some infrared and possibly other wavelengths of radiation that may be used to measure the amounts of certain substances.

U.S. Patent 5,693,944 (hereinafter "the '944 Patent"), issued to Rich on December 21, 1997, discloses a cuvette, a method for using the same, and a method for manufacturing the same. The cuvette and methods of use disclosed in the '944 Patent eliminate the problems that were previously encountered in attempts to use polymers in the place of sapphire windows. The '944 Patent discloses fashioning windows from a malleable homopolymer, such as biaxially oriented polypropylene, in the thickness range of 25 µm to 125 µm. The use of this inexpensive polypropylene material allows for the fabrication of single-use, disposable cuvettes.

### C. Luminescence Quenching and Fuel Cells

Luminescence quenching and fuel cells are techniques that have been used to measure oxygen concentrations in gases. In use of luminescence quenching to measure oxygen concentrations, a luminescable material is excited to luminescence. Upon exposure of the luminescing material to a gas mixture including oxygen, the luminescence is quenched, depending upon the amount (i.e., concentration or fraction) of oxygen to which the luminescable material is exposed, or the amount of oxygen in the gas mixture. Accordingly, the rate of decrease in the amount of luminescence, or quenching of luminescence, of the luminescable material (i.e., the amount of light emitted by the luminescable material) corresponds to the amount of oxygen in the gas mixture.

Typically, luminescence quenching requires the emission of excitation radiation from a source toward a luminescable material of a luminescence chemistry that may be quenched by, or is specific for, one or more types of gas (e.g., oxygen, carbon dioxide, halothane, etc.) to be measured. The excitation radiation causes the luminescable material to be excited and to emit electromagnetic radiation of a different wavelength than the excitation radiation. The presence of the one or more gases of interest quenches, or reduces, the amount of radiation emitted from the luminescable material. The amount of radiation emitted from the luminescable material is measured by a detector and compared with the amount of radiation emitted from the luminescable material in the absence of one or more quenching gases in order to facilitate a determination of the amount of the one or more sensed, quenching gases in the respiration of a patient.

A typical fuel cell include a gold cathode and a lead anode surrounded by an electrolyte. A membrane protects the cathode and anode. The gas to be monitored diffuses into the cell through the membrane. The oxygen causes an electro-chemical reaction in the fuel cell. As a result, the fuel cell generates and electric current in proportion to the partial pressure of the oxygen in the gas. Thus, the amount of current generated by the fuel cell indicates the concentration of oxygen in the gas being analyzed. An example of a mainstream gas monitoring system using a fuel cell is disclosed in U.S. patent application no. 10/494,273 (publication no. 2004/0267151).

Luminescence quenching and fuel cells have been used in a variety of applications, including in diagnostic techniques. The use of luminescence quenching or fuel cells in mainstream oxygen sensors has also been disclosed. Nonetheless, these mainstream sensors are not equipped to employ other gas monitoring techniques or to measure respiratory flow, severely limiting the functionality of these luminescence quenching and fuel cell type sensors.

### D. Respiratory Flow Monitoring

Respiratory flow measurement during the administration of anesthesia in intensive care environments and in monitoring the physical condition of athletes and other individuals prior to and during the course of training programs and medical tests provides valuable information for assessment of pulmonary function and breathing circuit integrity. Many different technologies have been applied to create a flow meter that meets the requirements of the critical care environment. Among the flow measurement approaches which have been used are:
a) Differential Pressure - measuring the pressure drop or differential across a resistance to flow (flow resistance);
b) Spinning Vane - counting the revolutions of a vane placed in the flow path;
c) Hot Wire Anemometer - measuring the cooling of a heated wire due to airflow passing around the wire;
d) Ultrasonic Doppler - measuring the frequency shift of an ultrasonic beam as it passes through the flowing gas;
e) Vortex Shedding - counting the number of vortices that are shed as the gas flows past a strut placed in the flow stream; and
f) Time of Flight - measuring the arrival time of an impulse of sound or heat created upstream to a sensor placed downstream.

Each of the foregoing approaches has various advantages and disadvantages, and an excellent discussion of most of these aforementioned devices may be found in W.J. Sullivan, G.M. Peters, P.L. Enright, M.D, "Pneumotachographs: Theory and Clinical Application", Respiratory Care, July 1984, Vol. 29-7, pp. 736-49, and in C. Rader, "Pneumotachography, a Report for the Perkin-Elmer Corporation" presented at the California Society of Cardiopulmonary Technologists Conference, October 1982.

At the present time, the most commonly used device for respiratory flow detection is the differential pressure flow meter. The relationship between flow and the pressure drop across a restriction or other resistance to flow is dependent upon the design of the resistance. Many different resistance configurations have been proposed. The goal of many of these configurations is to achieve a linear relationship between flow and pressure differential.

In some differential pressure flow meters, which are commonly termed "pneumotachs", the flow restriction has been designed to create a linear relationship between flow and differential pressure. Such designs include the Fleisch pneumotach in which the restriction is comprised of many small tubes or a fine screen to ensure laminar flow and a linear response to flow. Another physical configuration is a flow restriction having an orifice that varies in relation to the flow. This arrangement has the effect of creating a high resistance at low flows and a low resistance at high flows. Among other disadvantages, the Fleisch pneumotach is susceptible to performance impairment from moisture and mucous, and the variable orifice flow meter is subject to material fatigue and manufacturing variabilities.

Most all known prior art differential pressure flow sensors suffer deficiencies when exposed to less than ideal gas flow inlet conditions and, further, possess inherent design problems with respect to their ability to sense differential pressure in a meaningful, accurate, repeatable manner over a substantial dynamic flow range. This is particularly true when the flow sensor is needed to reliably and accurately measure low flow rates, such as the respiratory flow rates of infants.

U.S. Patent 5,379,650 (hereinafter "the '650 Patent"), issued to Kofoed et al. on January 10, 1995, has overcome the vast majority of the problems with differential pressure flow sensors with a sensor that includes a tubular housing containing a diametrically oriented, longitudinally extending strut. The strut of the flow sensor disclosed in the '650 Patent includes first and second lumens with longitudinally spaced pressure ports that open into respective axially located notches formed at each end of the strut.

Developments in patient monitoring over the past several decades have shown that concurrent measurements of various combinations of exhaled gas flow rate, O₂ concentrations, CO₂ concentrations, and concentrations of N₂O and various other anesthetic agents provide information that is useful in decision making with respect to anesthesia and therapy. By combining flow, airway pressure, CO₂, and O₂ measurements, one can calculate CO₂ elimination (VCO₂) and O₂ consumption (VO₂), which are related to the metabolic status of an individual. Also, these measurements can provide a graphical representation of the expired O₂ or CO₂ concentration versus expired volume which provides information about gas exchange in different compartments of the lungs.

While integrated adapters that include both flow and infrared CO₂ sensors are known, separate apparatus are presently necessary to obtain O₂ measurements and measurements of respiratory flow or of CO₂ or N₂O and other anesthetic agents. The various apparatus that are needed to simultaneously acquire a combination of the respiratory O₂ signals, respiratory flow signals, airway pressure signals, and signals representative of amounts of CO₂, N₂O, or anesthetic agents would require multiple components if such components were all available in a mainstream configuration. Such "stacking" of multiple sensors at the patient's airway is cumbersome and adds undesirable volume (dead space) and resistance to the breathing circuit. Patent application publication US US2002/029003 A1 discloses an integrated airway adapter capable of monitoring any combination of respiratory flow, O₂ concentration, and concentrations of one or more of CO₂, N₂O, and an anesthetic agent in real time, breath by breath.

It would be highly desirable to have an airway adapter which combines a luminescence quenching sensor with one or both of an infrared gas sensor and a respiratory flow sensor in a configuration which is convenient to use and which minimizes phase lag and internal dead space of the combination.

### DISCLOSURE OF THE INVENTION

The present invention is directed to a metabolic measurement system that includes an integrated airway adapter for monitoring, in real time, breath-by-breath amounts of substances, such as O₂, CO₂, N₂O, and anesthetic agents in the respiration of an individual, which includes normal respiratory gases, as well as other substances that are inhaled and exhaled by the individual as defined by claim 1. Advantageous embodiments are defined in the dependent claims. The airway adapter of the present invention is a compact adapter that integrates at least two functions into a single unit that meets the requirements for clinical patient monitoring. The airway adapter may include a combination of different types of substance detection components or a combination of one or more substance detection components and a respiratory flow detection component. From these measurements, metabolic parameters, such as oxygen consumption or oxygen uptake, carbon dioxide production or carbon dioxide elimination, respiratory quotient (RQ), resting energy expenditure (REE), or any combination of such measurements, can be determined.

In an exemplary embodiment of the present invention, the O₂ sensing portion of an integrated airway adapter, incorporating teachings of the present invention, includes a fuel cell or a quantity of luminescable material, the luminescence of which is quenched upon exposure to O₂, located in communication with a flow path along which respiratory gases are conveyed through the airway adapter so as to be exposed to the respiratory gases. The luminescable material of the O₂ sensing portion may be carried by a removable, replaceable portion of the airway adapter to facilitate reuse of the airway adapter. A source of excitation radiation may be configured to be coupled to the airway adapter so as to direct radiation through a window of the airway adapter and toward the luminescable material to excite the same to luminesce, or to emit radiation. The amount of radiation emitted from the excited luminescable material may be measured with a detector, which may also be configured for assembly with the airway adapter, which detects emitted radiation through a window of the airway adapter.

The present invention further contemplates that the integrated airway adapter also includes a flow sensor. In one embodiment, the flow sensor is a pneumotach that includes two pressure ports, which facilitate the generation of a differential pressure across an orifice of the pneumotach. One of the pressure ports may facilitate monitoring of airway pressure. Alternatively, the flow sensor may have more than two ports, with at least one of the ports facilitating measurement of the airway pressure. The respiratory flow sensor preferably has the capability of accommodating a wide variety of gas flow inlet conditions without adding significant system volume or excessive resistance to the flow of respiration through the integrated airway adapter of the present invention. The design of the respiratory flow sensor of the present invention may also substantially inhibit the introduction of liquids into the pressure ports or monitoring system of the sensor.

The flow sensor may include a flow resistance element (whether the strut or the gas concentration monitoring portion) which creates a nonlinear differential pressure signal. To obtain adequate precision at extremely high and low flow rates, a very high resolution (e.g., 18-bit or 20-bit) analog-to-digital (A/D) conversion device may be used. The use of such a very high resolution A/D converter allows a digital processor to compute flow from the measured differential pressure by using a sensor characterizing look-up table. This technique eliminates the need for variable or multiple gain amplifiers and variable offset circuits that might otherwise be required with use of a lower resolution A/D converter (e.g., a 12-bit A/D converter).

Alternatively, or in addition to the flow sensor, an integrated airway adapter incorporating teachings of the present invention may include a gas sensor configured to measure amounts of CO₂, N₂O, or anesthetic agents in the respiration of an individual. As an example, the airway adapter may include a gas sensor that employs infrared absorption techniques. Such an exemplary gas sensor may include a chamber with a pair of opposed, substantially axially aligned windows flanking a flow path through the airway adapter. The windows preferably have a high transmittance for radiation in at least the intermediate infrared portion of the electromagnetic spectrum. It is essential to the accuracy of the infrared gas sensor that the material used for the windows transmit a usable part of the infrared radiation impinging thereupon. Thus, the window material must have appropriate optical properties. Preferred window materials include, but are not limited to, sapphire and biaxially oriented polypropylene. Substantial axial alignment of the windows allows an infrared radiation beam to travel from a source of infrared radiation, transversely through the chamber and the gas(es) flowing through the chamber, to an infrared radiation detector. Alternatively, the airway adapter may include a single window and a reflective element, such as a mirror or reflective coating. These elements facilitate the direction of infrared radiation into and across the chamber and the reflection of the infrared radiation back across and out of the chamber to a radiation detector. Signals from the detector facilitate determination of the amounts (i.e., concentrations or fractions) of one or more gases, such as CO₂, N₂O, and anesthetic agents, in respiration flowing through the chamber.

The integrated airway adapter can be either reusable or disposable. If the airway adapter is designed to be disposable, the infrared absorption windows and the windows that facilitate detection of luminescence quenching should be made of an inexpensive material. If the airway adapter is designed to be reused, the windows of the infrared gas sensor may be detachable from the remainder of the airway adapter so as to facilitate the cleaning and sterilization of nondisposable windows. Alternatively, the windows may remain on the airway adapter during cleaning and sterilization thereof. If luminescable material is carried upon any portion of one or both windows, the luminescable material may be removed from the windows during cleaning and subsequently replaced or, if the luminescable material will withstand the cleaning and sterilization processes, the luminescable material may remain on the windows during these processes.

Injection molding processes may be used to manufacture the airway adapter of the present invention. The consistency of product obtainable from the injection molding process provides a high degree of interchangeability, thereby eliminating the need for a calibration procedure to be performed during setup or with a disposable adapter replacement.

In addition, the integrated airway adapter may incorporate a specific instrument connection scheme to facilitate the proper assembly of external components (e.g., an infrared emitter and detector, a luminescence quenching source and detector, etc.) with the airway adapter, as well as to facilitate the proper assembly of the airway adapter with a respiratory airway. For example, but not to limit the scope of the present invention, the airway adapter may include colors, optical coding, or other suitable types of coding to facilitate correct assembly or may be configured so as to prevent improper assembly.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictates otherwise.

### BRIEF DESCRIPTION OF THE DRAWINGS

FIG. 1 is an exploded perspective view of a first preferred embodiment of the airway adapter of the present invention in combination with a transducer housing for containing electronics for respiratory and anesthetic agent gas determination;
FIG. 2 is a side elevation view of a first preferred embodiment of the airway adapter of the present invention;
FIG. 2A is top elevation view of a first preferred embodiment of the airway adapter of the present invention;
FIG. 3 is an end elevation view of the airway adapter of FIG. 2, looking from plane 3-3;
FIG. 4 is a side sectional elevation view of the airway adapter of FIG. 2;
FIG. 5 is a sectional view of the airway adapter of FIG. 4, looking upward from plane 5-5 extending laterally across the axis of the airway adapter of the present invention;
FIG. 6 is another sectional elevation view of the airway adapter of FIGS. 2 and 4, looking from plane 6-6 of FIG. 4, and schematically illustrating a transducer assembled therewith;
FIG. 7 is a cross-sectional representation of an airway adapter that includes a single window through which a luminescence quenching measurement of one or more substances may be obtained and a pair of opposed windows through which an infrared measurement of one or more substances may be obtained;
FIG. 8 is a cross-sectional representation of an airway adapter that includes a single window through which a luminescence quenching measurement of one or more substances may be obtained and another single window and corresponding optics through which an infrared measurement of one or more substances may be obtained;
FIGS. 9 and 11 are cross-sectional assembly views of alternative embodiments of airway adapters and transducers according to the present invention, which include pairs of opposed windows through which both luminescence quenching and infrared measurements of one or more substances may be obtained;
FIGS. 10 and 12 are partial views of airway adapter windows of the airway adapter embodiments depicted in FIGS. 9 and 11, respectively;
FIG. 13 is a cross-sectional representation of the airway adapter that includes a single window through which both infrared and luminescence quenching measurements may be taken;
FIG. 14 is a cross-section taken along line 14-14 of FIG. 13, also showing a transducer assembled with the airway adapter;
FIG. 15 is a side elevation view of a second preferred embodiment of the airway adapter of the present invention;
FIG. 16 is a side elevation view of a third preferred embodiment of the airway adapter of the present invention;
FIG. 17 is a side sectional elevation of the airway adapter of FIG. 16;
FIG. 18 is a bottom view of the airway adapter of FIG. 16;
FIG. 19 is a side elevation view of a fourth preferred embodiment of the airway adapter of the present invention;
FIG. 20 is a side sectional elevation of the airway adapter of FIG. 19;
FIG. 21 is an end elevation view of the airway adapter along lines 21-21 of FIG. 19;
FIG. 22 is an end elevation view of the airway adapter along lines 22-22 of FIG. 19;
FIG. 23 is a sectional view of the airway adapter of FIG. 19, looking from plane 23-23;
FIG. 24 is a sectional view of the airway adapter of FIG. 19, looking from plane 24-24;
FIG. 25 is a sectional view of the airway adapter of FIG. 19, looking from plane 25-25;
FIG. 26 is a sectional view of the airway adapter of FIG. 19, looking from plane 26-26;
FIG. 27 is a schematic view of a first embodiment of a metabolic measurement system according to the principles of the present invention;
FIG. 28 is a schematic view of a second embodiment of a metabolic measurement system according to the principles of the present invention;
FIG. 29 is a schematic view of a third embodiment of a metabolic measurement system according to the principles of the present invention;
FIG. 30 is a schematic view of a fourth embodiment of a metabolic measurement system according to the principles of the present invention;
FIG. 31 is a schematic view of a fifth embodiment of a metabolic measurement system according to the principles of the present invention; and
FIG. 32 is a schematic view of an airway adapter combined with a flow measurement system.

### DETAILED DESCRIPTION OF THE EXEMPLARY EMBODIMENTS

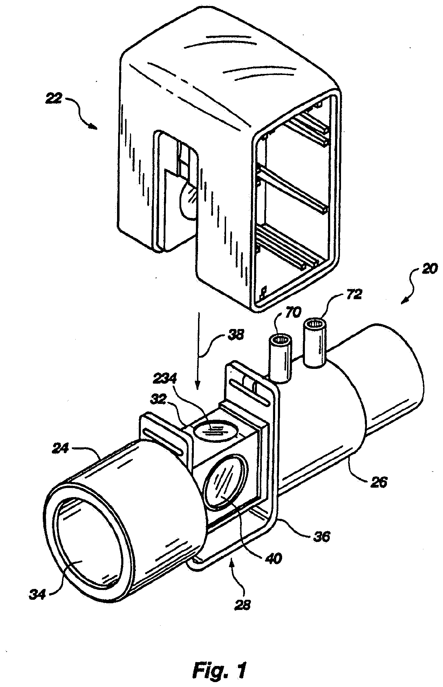

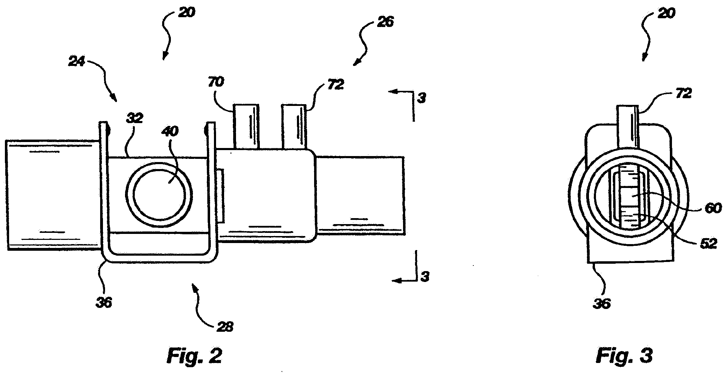

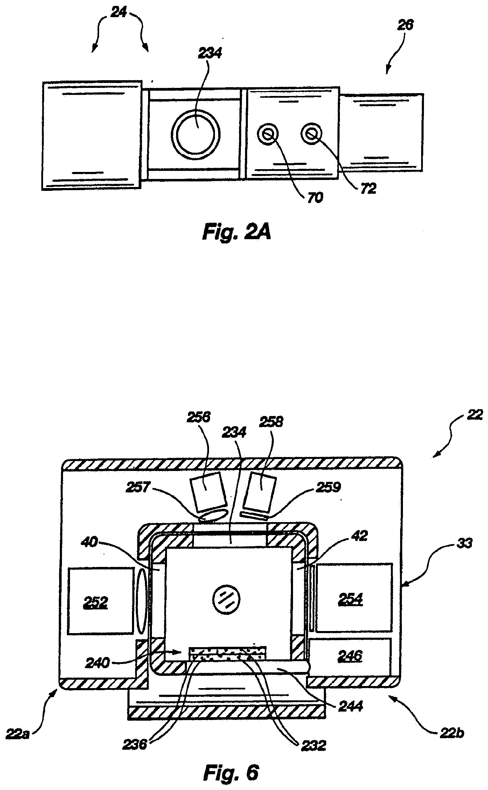

FIGS. 1-5 illustrate an exemplary airway adapter 20 embodying teachings of the present invention. Airway adapter 20 is preferably a unitary, injection-molded plastic element, so as to afford low manufacturing cost and permit disposal of the sensor after a single use, with a separate transducer housing 22 containing an infrared emitter 252, an infrared detector 254, a luminescence excitation radiation source 256, and a luminescence detector 258 (FIG. 6). However, this configuration is not a requirement. As illustrated, airway adapter 20 has a generally parallelepipedal center section 32 between and axially aligned with first and second tubular portions 24 and 26, with a flow passage 34 extending from end-to-end through airway adapter 20.

The illustrated airway adapter 20 is designed for connection with a breathing circuit that communicates with the airway of a patient. Airway adapter 20 may be connected between a patient ventilation device and the tubing of a mechanical ventilator. For example, first tubular portion 24 of airway adapter 20 may be connected to an endotracheal tube inserted in the trachea of a patient, while second tubular portion 26 of airway adapter 20 is attached to the tubing of the mechanical ventilator. Alternatively, airway adapter 20 may be connected to a breathing mask or other apparatus that are less invasive than endotracheal tubes. Airway adapter 20 need not be connected to a mechanical ventilator, but may be connected with a source of respiratory gases (e.g., an oxygen source) or communicate directly with the air from the patient's environment. As shown, first and second tubular portions 24 and 26 have bores of varying diameter and substantially circular cross-sections, with a gas concentration monitoring portion 28 disposed therebetween. Second tubular portion 26 houses a respiratory flow monitoring device 30.

Gas concentration monitoring portion 28 includes a gas sensing portion 230, which is configured to employ luminescence quenching techniques to measure the partial pressure or amount of oxygen or other gases that flow through airway adapter 20. As illustrated in FIGS. 1, 2A, 4 and 5, gas sensing portion 230, also referred to as "gas sensor 230," includes a quantity of luminescable material 232 exposed to a flow passage 34 that extends through airway adapter 20. Gas sensing portion 230 also includes a window 234 for facilitating the excitation of luminescable material 232 or some combination of luminescable materials with radiation of one or more excitation wavelengths, as well as the measurement of the intensities of one or more wavelengths of radiation that are emitted from luminescable material 232, as illustrated in FIGS. 1, 2A, and 4. Window 234 preferably has a high transmittance for wavelengths of excitation radiation, which excite luminescable material 232, and for wavelengths of radiation emitted from luminescable material 232.

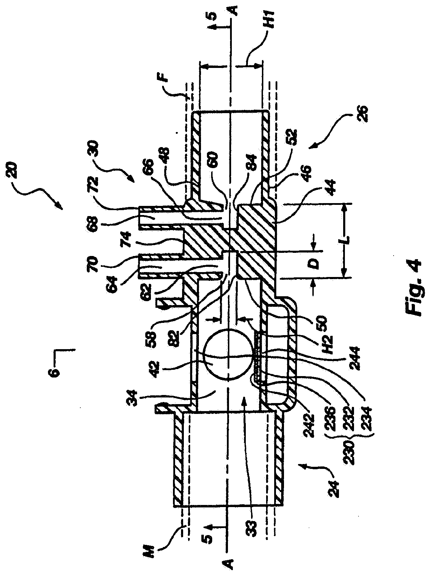

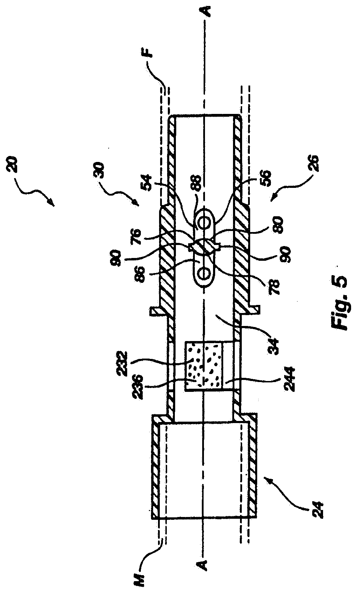

With specific reference to FIGS. 4 and 5, luminescable material 232 is preferably carried by a membrane 236, or matrix, which is disposed on or comprises an integral part of a surface of flow passage 34. Alternatively, a membrane 236 carrying luminescable material 232 may be located in another portion of airway adapter 20 that communicates with flow passage 34.

Luminescable material 232 may be dispersed throughout passages or openings formed in membrane 236. The passages and openings through membrane 236 may have diameters or widths of about 0.1 µm to about 10 µm, as the diffusion constant for molecular oxygen through membranes of such dimensions is large enough to provide a luminescence quenching response time of sufficiently short duration to facilitate a measurement of the luminescence quenching rate on a breath-by-breath basis, or in real time. Stated another way, these membrane 236 dimensions facilitate the substantially immediate exposure of luminescable material 232 to oxygen and other luminescence quenching substances as these substances flow through or past membrane 236.

If airway adapter 20 is reusable, membrane 236 may be removable from the remainder of airway adapter 20 so as to facilitate replacement thereof with a new membrane 236 carrying luminescable material 232 and, thus, to facilitate accurate determinations of the concentration of oxygen or other gases with subsequent use of airway adapter 20. Alternatively, if luminescable material 232 will withstand the cleaning and sterilization processes to which airway adapter 20 is subjected, membrane 236 may be permanently secured to airway adapter 20 and reused following cleaning and sterilization thereof.

Porphyrins are an example of a material that may be used as luminescable material 232. Porphyrins are stable organic ring structures that often include a metal atom. When the metal atom is platinum or palladium, the phosphorescence decay time ranges from about 10 µs to about 1,000 µs. Porphyrins are also sensitive to molecular oxygen. When porphyrins are used as luminescable material 232, it is preferred that the porphyrins retain substantially all of their photo-excitability with repeated use. Stated another way, it is preferred that the porphyrins be "photostable". Fluorescent porphyrins, such as meso-tetraphenyl porphines, are particularly photostable. The various types of porphyrins that may be used as luminescable material 232 to facilitate oxygen detection include, without limitation, platinum meso-tetra(pentafluoro)phenyl porphine, platinum meso-tetraphenyl porphine, palladium meso-tetra(pentafluoro)phenyl porphine, and palladium meso-tetraphenyl porphine. Of course, other types of luminescable materials that are known to be quenched upon being exposed to oxygen, carbon dioxide, or another analyzed substance (e.g., gas, liquid, or vapor) may also be used in airway adapters incorporating teachings of the present invention.

Membrane 236 is preferably formed from a material that is compatible with luminescable material 232. Moreover, it is preferred that the material of membrane 236 be compatible with respiratory gases, as well as nontoxic to the patient and, preferably, to the environment.

Materials that may be used to form membrane 236 include, but are not limited to, porous polyvinylchloride (PVC), polypropylene, polycarbonate, polyester, polystyrene, polymethacrylate polymers, and acrylic copolymers. Specifically, microporous polycarbonate filtration membranes available from Pall Gelman Sciences of Ann Arbor, Michigan, and from Whatman, Inc. of Clifton, New Jersey, (track-etched microporous polycarbonate filtration membranes with a thickness of about 10 µm and a pore size of about 0.4 µm) are useful as membrane 236.

As indicated previously herein, it is preferred that membrane 236 be permeable to respiratory gases, including oxygen. As respiratory gases flow past, into, or through membrane 236, the respiratory gases, including oxygen, contact luminescable material 232 carried thereby. The luminescence of, or intensity of radiation emitted from, luminescable material 232 is then quenched to a degree that is based on the amount of oxygen or other luminescence quenching gases in the respiratory gases. The permeability of membrane 236 to respiratory gases also has an effect on the number of luminescable material 232 particles that is exposed to the respiratory gases and may, therefore, affect the amount of luminescence quenching that occurs as luminescable material 232 is exposed to oxygen and other luminescence quenching gases present in the respiratory gases that flow through membrane 236.

Luminescable material 232 may be applied to membrane 236 by known processes. By way of example and not to limit the scope of the present invention, a solvent may be used to introduce luminescable material 232 onto a surface of membrane 236, as well as into openings thereof. Preferably, the solvent does not substantially dissolve the material of membrane 236. The solvent may, however, interact with the material of membrane 236 in a manner that causes membrane 236 and the openings thereof to swell, so as to facilitate the introduction of luminescable material 232 into the openings. Exemplary solvents that may be used to apply luminescable material 232 to membrane 236 include, without limitation, hexane, petroleum ethane, toluene, tetrahydrofuran, methylene chloride, trichloroethylene, xylene, dioxane, isopropyl alcohol, and butanol, as well as mixtures of any of the foregoing. Of course, the use of a particular solvent depends on its compatibility with both luminescable material 232 and with the material of membrane 236. Once luminescable material 232 has been applied to membrane 236, the solvent may be evaporated or otherwise removed from membrane 236 in a manner that leaves luminescable material 232 on the surface and within the openings of membrane 236.

Alternatively, as shown in FIG. 6, luminescable material 232 may be sandwiched between two membranes 236. A solvent that will not significantly degrade luminescable material 232 dissolves the material of membranes 236 enough to bond membranes 236 to one another to form a single composite membrane 240, but without substantially altering the structures of membranes 236. Luminescable material 232 remains between membranes 236 and may at least partially permeate membranes 236. As membranes 236 trap luminescable material 232 therebetween, increased concentrations of luminescable material 232 may be incorporated into composite membrane 240 relative to the concentration of luminescable material 232 contained by a single membrane 236.

With returned reference to FIG. 4, sensor 230 may include an overcoat layer 242 over membrane 236. Overcoat layer 242 may be formed from a polymer, such as the same type of polymer from which membrane 236 is formed, or from a different type of polymer than that from which membrane 236 is formed. Overcoat layer 242 does not substantially prevent gases in the respiration of an individual from contacting luminescable material 232. Overcoat layer 242 may also refine or tailor various properties of membrane 236, including, without limitation, the light absorption properties of membrane 236, the light transmission properties of membrane 236, and the permeability of membrane 236 to various gases. As an example of the use of an overcoat layer 242 to tailor the properties of membrane 236, permeability of membrane 236 to oxygen or other respiratory gases may be reduced by applying to membrane 236 an overcoat layer 242 formed from a less permeable material.

Known processes may be used to apply overcoat layer 242 to membrane 236. For example, a dissolved polymer may be applied to membrane 236 to form overcoat layer 242. Alternatively, a preformed overcoat layer 242 may be adhered to membrane 236 by known means, so long as the overcoated membrane 236 retains the desired properties.

In use of gas sensor 230, membrane 236 thereof is preferably disposed over a thermal source of a known type, such as thermal capacitor 244. Thermal capacitor 244 communicates with a heater component 246 (FIG. 6), which heats thermal capacitor 244 to a desired, substantially constant temperature. Because thermal capacitor 244 contacts membrane 236, thermal capacitor 244, in turn, heats membrane 236 to a substantially constant temperature. Accordingly, thermal capacitor 244 substantially prevents temperature changes of membrane 236 or of luminescable material 232 thereon from affecting the luminescence quenching caused by oxygen or other substances flowing past luminescable material 232.

One example of the manner in which thermal capacitor 244 and heater component 246 may communicate with each other includes providing a floating, thermally conductive heater component 246 on transducer housing 22 (FIG. 6). Upon coupling transducer housing 22 with airway adapter 20, heater component 246 and thermal capacitor 244 contact one another in such a manner as to provide an efficient transfer of heat from heater component 246 to thermal capacitor 244.

Transducer housing 22, as depicted in FIG. 6, at least partially contains a radiation source 256, which emits electromagnetic excitation radiation of one or more wavelengths that will excite luminescable material 232 into luminescence. For example, radiation source 256 may comprise a light-emitting diode (LED), which produces excitation radiation in the form of visible light. Radiation source 256 preferably emits excitation radiation of wavelengths that will excite luminescable material 232 to emit a desired intensity of radiation. Excitation radiation emitted from radiation source 256 passes through and is focused by a lens 257, which directs the focused excitation radiation toward luminescable material 232.

Transducer housing 22 also contains at least a portion of a detector 258 positioned to receive radiation emitted from luminescable material 232 and configured to measure an intensity of such emitted radiation. Accordingly, detector 258 is positioned toward window 234 and toward luminescable material 232. Preferably, a filter 259 is disposed between luminescable material 232 and detector 258 so as to prevent wavelengths of electromagnetic radiation other than those emitted from luminescable material 232 from interfering with the luminescence and luminescence quenching measurements obtained with detector 258. Other features and advantages of a luminescence quenching type sensor that may also be employed in the present invention is disclosed in U.S. Patent 6,325,978, issued to Labuda et al. on December 4, 2001, which has been assigned to the same assignee as the present invention.

Gas concentration monitoring portion 28 of airway adapter 20 provides a seat for transducer housing 22. An integral, U-shaped casing element 36 positively locates transducer housing 22 across airway adapter 20 and in the transverse direction indicated by arrow 38 in FIG. 1. Arrow 38 also shows the direction in which transducer housing 22 is displaced to detachably assemble it to airway adapter 20. In a preferred embodiment, transducer housing 22 snaps into place on airway adapter 20, as disclosed in the '858 and '859 Patents; no tools are needed to assemble airway adapter 20 and transducer housing 22 or to remove transducer housing 22 from airway adapter 20.

Center section 32 may also include an infrared sensor portion 33 with first and second axially aligned windows 40 and 42, respectively (only window 42 is shown in FIG. 4). Windows 40 and 42 preferably have a high transmittance for radiation in at least the intermediate infrared portion of the electromagnetic spectrum. The substantial axial alignment of first window 40 and second window 42 allows an infrared radiation beam to travel from infrared emitter 252 in one leg 22a of transducer housing 22, transversely through airway adapter 20 and the one or more gases flowing through flow passage 34 of airway adapter 20, to infrared detector 254 in the opposing, substantially parallel leg 22b of transducer housing 22.

Cuvette windows 40 and 42 for infrared absorption measurements have typically been fabricated from sapphire because of sapphire's favorable optical properties, stability, and resistance to breakage, scratching, and other forms of damage. Alternatively, the cost of the cuvette can be reduced to the point of making it practical to dispose of the cuvette after a single use by fabricating the cuvette windows from an appropriate polymer. It is essential to the accuracy of the infrared absorption portion of the gas concentration monitor that the polymer transmit a usable part of the infrared radiation impinging upon it. Thus, the window material must have the appropriate optical properties for measuring the desired substances. An exemplary window material exhibiting such properties with respect to measuring an amount of carbon dioxide present in the respiration of a patient is biaxially oriented polypropylene. Other materials may also be used, depending upon the transmissivities thereof for certain wavelengths of radiation that are to be used to detect the presence or amounts of particular substances in the respiration of a patient.

Referring again to FIGS. 1 and 6, a transducer housing 22 is illustrated which carries electronic components that are designed to facilitate the output of one or more reference signals and one or more signals related to the concentrations of corresponding respiratory or anesthetic gases flowing through airway adapter 20. An infrared emitter 252 of transducer housing 22 is configured to direct infrared radiation of one or more wavelengths into center section 32 of airway adapter 20 through window 40, through a sample of respiratory gases within center section 32, and out of center section 32 through window 42. Infrared detector 254, which is positioned adjacent window 42 when transducer housing 22 is assembled with airway adapter 20, is positioned to receive infrared radiation signals that exit center section 32 of airway adapter 20 through window 42.

The internal configuration and design of infrared detector 254, which preferably monitors, in real time, the amounts of CO₂, N₂O, or anesthetic agents in the respiration of an individual is thoroughly discussed in U.S. Patent 5,616,923 (hereinafter "the '923 Patent"). It is understood that infrared CO₂ monitor devices such as those disclosed in the '858, '859, and '436 Patents, as well as other CO₂ detection devices, could be used in transducer housing 22. In addition to one or more infrared sensors, infrared detector 254 may include any combination of other components, including a reference sensor, optics (e.g., lenses, filters, mirrors, beam splitters, etc.), coolers, and the like.

The infrared signals detected by infrared detector 254 can be ratioed to provide a signal accurately and dynamically representing the amount of CO₂, N₂O, or an anesthetic agent flowing through airway adapter 20.

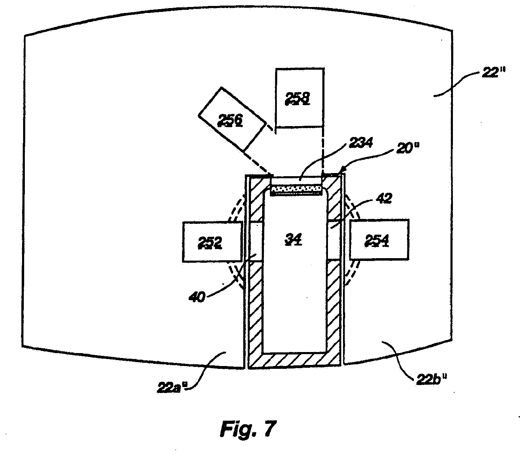

FIG. 7 illustrates another embodiment of airway adapter 20" and of a complementary transducer housing 22" assembled therewith.

Airway adapter 20" includes a window 234 formed through a top portion thereof. Window 234 is transparent to (i.e., has a high transmissivity for) wavelengths of radiation that are used to excite luminescable material 232 on a membrane 236 positioned within flow passage 34 and adjacent to window 234. In addition, window 234 is transparent to one or more wavelengths of radiation that are emitted from luminescable material 232 and quenched by an analyzed substance to a degree that relates to an amount of the analyzed substance in respiration of an individual or in another gas mixture.

In addition, airway adapter 20" includes windows 40, 42 positioned on opposite sides of flow passage 34. Windows 40 and 42 facilitate the direction of radiation of one or more specified infrared wavelengths across flow passage 34 to facilitate the measurement of amounts of one or more substances, such as carbon dioxide or nitrous oxide or other anesthetic agents, that are present in the respiration of an individual as the individual's respiration passes through a location of flow passage 34 between which windows 40 and 42 are positioned. Accordingly, windows 40 and 42 are each preferably formed from a material that is substantially transparent to (i.e., has a high transmissivity for) infrared wavelengths that are desired for use in measuring amounts of one or more substances in respiration of the individual.

Transducer housing 22" contains at least a portion of a radiation source 256 positioned to direct one or more wavelengths of radiation that are capable of exciting luminescable material 232 into luminescence through window 234, toward luminescable material 232. Radiation source 256 may include optics (e.g., filters, lenses, beam splitters, etc.) that direct radiation toward the appropriate location and that filter out one or more undesirable wavelengths of the radiation emitted from radiation source 256. In addition, transducer housing 22" carries a luminescence detector 258, as well as any optics (e.g., filters, lenses, beam splitters, etc.) associated therewith, which are respectively positioned to receive and detect at least one wavelength of radiation that is emitted by luminescable material 232 and that is quenched by exposure to a substance of interest to a degree that indicates an amount of the substance to which luminescable material 232 is exposed.

An infrared emitter 252 and an infrared detector 254 are positioned in opposite legs 22a", 22b", respectively, of transducer housing 22". Infrared emitter 252 is oriented within transducer housing 22" so as to direct one or more infrared wavelengths of radiation through window 40, across flow passage 34, and through window 42 as transducer housing 22" is assembled with airway adapter 20". Infrared detector 254, which is positioned adjacent window 42 when transducer housing 22" is assembled with airway adapter 20", is oriented so as to receive and detect the one or more infrared wavelengths of radiation emitted by radiation source 256 that exit airway adapter 20" through window 42.

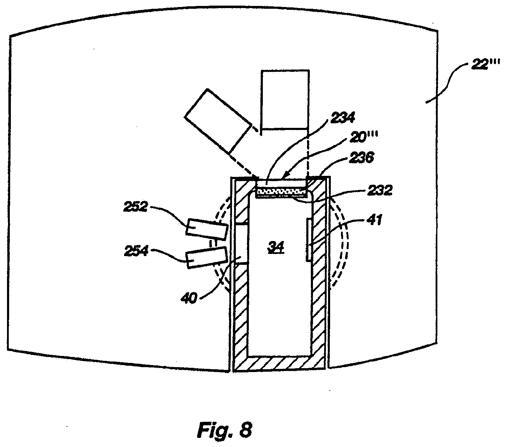

Alternatively, or in combination with other airway adapter features disclosed herein, as depicted in FIG. 8, an airway adapter 20 incorporating teachings of the present invention includes a single window 40 through which an infrared emitter 252 and infrared detector 254 may be used to measure an amount of a substance, such as carbon dioxide, nitrous oxide or another anesthetic agent, in the respiration of an individual. Window 40 of airway adapter 20 is positioned on one side of flow passage 34 to facilitate the introduction of one or more infrared wavelengths of radiation into flow passage 34, while optics 41, which reflect or otherwise redirect infrared wavelengths of radiation back across flow passage 34 and through window 40, are positioned at least partially across flow passage 34 from window 40.

Window 40 may be formed from a material that is substantially transparent to (i.e., has a high transmissivity for) infrared wavelengths that are desired for use in measuring amounts of one or more substances in respiration of the individual.

Optics 41 may include one or more mirrors or reflective coatings, as well as other optical components of known types (e.g., lenses, filters, etc.), to direct a beam of radiation that originated from an infrared emitter 252 within transducer housing 22 and was introduced into flow passage 34 of airway adapter 20 back across flow passage 34, through window 40, and to an infrared detector 254 carried by transducer housing 22, positioned adjacent infrared emitter 252.

As in previously described embodiments, airway adapter 20 is configured to seat a transducer housing 22, which carries infrared emitter 252 and infrared detector 254. Upon assembling transducer housing 22 and airway adapter 20, infrared emitter 252 is oriented such that it is positioned to emit infrared wavelengths of radiation into window 40, at least partially across flow passage 34, toward optics 41. Likewise, upon assembling airway adapter 20 and transducer housing 22, infrared detector 254 is oriented so as to receive infrared wavelengths of radiation that have been redirected by optics 41 back out of window 40.

As one or more infrared wavelengths of radiation pass across at least a portion of flow passage 34 adjacent to window 40 and through the respiration of an individual passing through that portion of flow passage 34, each infrared wavelength may be attenuated, or decreased in intensity, to a degree that correlates to an amount of a corresponding substance present in the individual's respiration.

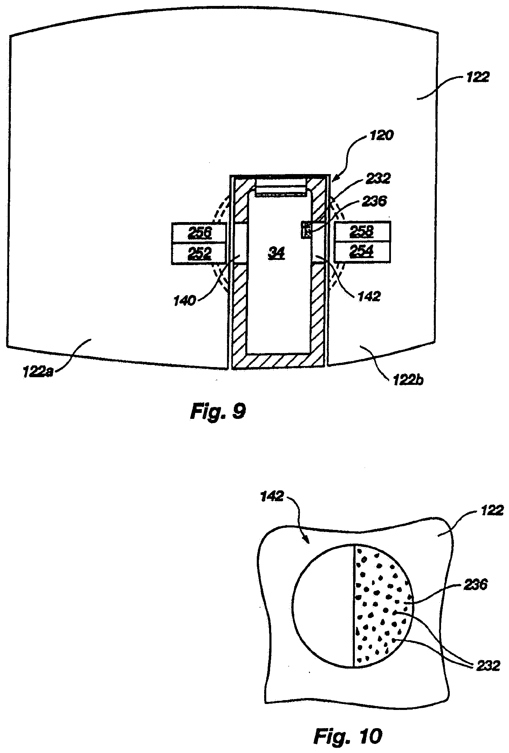

Other exemplary embodiments of airway adapters incorporating teachings of the present invention are depicted in FIGS. 9-12. As shown in FIGS. 9-12, an airway adapter 120 of the present invention may include a single pair of windows 140 and 142 through which both infrared and luminescence quenching measurements may be obtained.

Window 140 is substantially transparent to (i.e., has a high transmissivity for) at least one wavelength of radiation that excites luminescable material 232 into luminescence. In addition, window 140 is substantially transparent to one or more of infrared wavelengths of radiation that are useful for measuring amounts of one or more substances present in respiration or other gas mixtures passing through a location of flow passage 34 positioned between windows 140 and 142.

Window 142 is substantially transparent to the one or more infrared wavelengths of radiation to which window 140 is substantially transparent. Window 142 is also substantially transparent to at least one wavelength of radiation that is emitted by luminescable material 232, the intensity of which decreases at a rate that is indicative of an amount of a measured substance in respiration within flow passage 34.

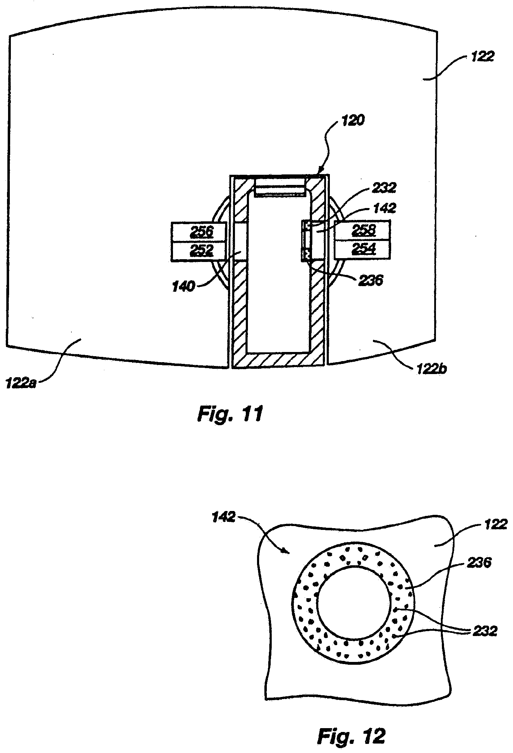

While radiation may pass through any portion of window 140, a membrane 236 carrying luminescable material 232 is positioned adjacent to a portion of window 142. As shown in FIGS. 9 and 10, membrane 236 is semicircular in shape. FIGS. 11 and 12 depict a membrane 236 having an annular shape and positioned adjacent an outer periphery of window 142. Membranes 236 of other shapes and covering different portions of window 142 are also within the scope of the present invention.

A transducer housing 122 configured complementarily to airway adapter 120 includes two legs 122a and 122b, one of which (first leg 122a) is configured to be positioned adjacent to window 140 and the other of which (second leg 122b) is configured to be positioned adjacent to window 142.

First leg 122a of transducer housing 122 carries infrared emitter 252 and radiation source 256, which emits at least one wavelength of radiation that will excite luminescable material 232. Both infrared emitter 252 and radiation source 256 are positioned to emit their respective wavelengths of radiation into window 140 and through flow passage 34. While infrared emitter 252 is also oriented so as to direct radiation emitted therefrom through an unobstructed (by membrane 236) portion of window 142, radiation source 256 is oriented to direct radiation emitted therefrom toward membrane 236 so as to excite luminescable material 232 carried thereby into luminescence.

As an alternative, membrane 236 may substantially cover window 142 if membrane 236 and luminescable material 232 thereon are substantially transparent to one or more wavelengths of infrared radiation that are used to detect the partial pressure or amount of carbon dioxide or one or more other substances present in respiratory or other gases that are flowing through airway adapter 120.

Second leg 122b of transducer housing 122 carries an infrared detector 254 and luminescence detector 258. Infrared detector 254 is positioned to receive and detect one or more infrared wavelengths of radiation exiting airway adapter 120 through window 142. Luminescence detector 258 is oriented to receive and detect one or more wavelengths of radiation that are emitted from luminescable material 232 and that are quenched, or reduced in intensity, to a degree representative of an amount of a monitored substance in respiration to which luminescable material 232 is exposed.

As an alternative to the embodiments illustrated in FIGS. 9 and 11, radiation source 256 may be located within second leg 122b of transducer housing 122 and positioned to direct radiation toward a portion of window 142 adjacent to which membrane 236 with luminescable material 232 thereon is positioned. As another alternative, one or both of luminescence detector 258 and radiation source 256 could be carried by first leg 122b of transducer housing 122.

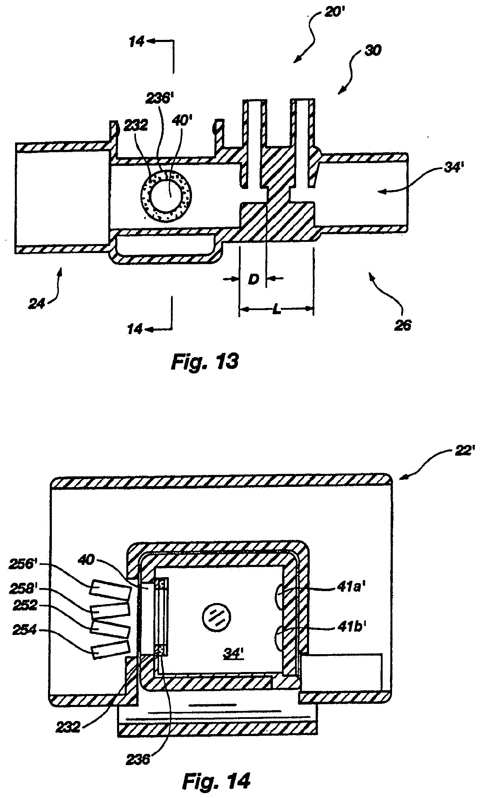

FIGS. 13 and 14 depict another exemplary embodiment of airway adapter 20' incorporating teachings of the present invention, which includes a single window 40' through which measurements of the amounts of oxygen, carbon dioxide, and anesthetic agents in the respiration of an individual may be obtained. As illustrated, membrane 236', which carries luminescable material 232, is positioned within flow passage 34' on a portion of window 40'. While membrane 236' is depicted as being annular in shape and covering a periphery of window 40', airway adapters with other shapes of membranes are also within the scope of the present invention. Furthermore, the membrane that carries luminescable material 232 need not be positioned on window 40', but may be positioned elsewhere within flow passage 34' or in a location that is in flow communication with flow passage 34'.

Airway adapter 20' also includes one or more mirrors 41' that are positioned so as to facilitate measurement of the amounts of one or more of oxygen, carbon dioxide, and anesthetic agents in the respiration of an individual through window 40'. As depicted, airway adapter 20' includes one mirror 41', which facilitates collection of measurements that are indicative of an amount of carbon dioxide and/or an anesthetic agent in an individual's respiration. By way of example only, mirror 41' may be shaped or positioned within flow passage 34 so as to reflect radiation that has been introduced into flow passage 34 through window 40' and that has traversed at least a portion of the distance across flow passage 34 back through window 40'. Of course, mirror 41' may actually comprise a group of mirrors or other optical elements (e.g., filters, lenses, etc.) or known types to facilitate the direction of radiation of particular wavelengths to the appropriate locations.

As depicted in FIG. 14, a transducer housing 22' that is configured to be assembled with airway adapter 20' includes a radiation source 256 and a corresponding luminescence detector 258. Radiation source 256 emits at least one wavelength of electromagnetic radiation that will excite luminescable material 232. Radiation source 256 is positioned to introduce one or more wavelengths of excitation radiation through window 40' and onto luminescable material 232. At least a portion of the radiation that is emitted from luminescable material 232 is then received by luminescence detector 258. Luminescence detector 258 detects at least one wavelength of radiation emitted from luminescable material 232 that indicates an amount of oxygen present in respiration or another gas mixture flowing through flow passage 34.

Transducer housing 22', as shown in FIG. 14, may also carry an infrared emitter 252 and an infrared detector 254. Infrared emitter 252 emits one or more wavelengths of radiation that are useful for detecting an amount of carbon dioxide, an anesthetic agent, or another gas or vaporized material that is present in respiration or another mixture of gases located within flow passage 34'. As shown, infrared emitter 252 is positioned to direct the one or more wavelengths of radiation into window 40', at least partially across flow passage 34', and toward mirror 41'. Mirror 41' then reflects the one or more wavelengths of radiation back toward a location of window 40' where the radiation will be received or sensed by infrared detector 254.

Of course, one or more lenses may be associated with radiation source 256' and/or luminescence detector 258' to focus radiation being emitted by radiation source 256' or received by luminescence detector 258'. One or more filters may similarly be associated with radiation source 256' to limit the wavelengths of radiation to which luminescable material 232 is exposed. Also, one or more filters may be associated with luminescence detector 258' to restrict the wavelengths of radiation that maybe received thereby.

Referring generally to FIGS. 1-5, 13, and 14 airway adapter 20, 20' and transducer housing 22, 22' may be molded from a polycarbonate or a comparable rigid, dimensionally stable polymer. Nonetheless, several factors, including, without limitation, the type of luminescable material 232 being used, as well as wavelengths of radiation that excite luminescable material 232, that are emitted by luminescable material 232, and that are used to detect other substances, such as carbon dioxide or nitrous oxide or other anesthetic agents, may also be taken into consideration when selecting the material or materials that are to be used to form airway adapter 20, 20'. Such factors may also be considered when selecting one or more materials from which transducer housing 22, 22' will be formed.

When an airway adapter 20, 20' incorporating teachings of the present invention includes luminescable material 232, the material or materials from which airway adapter 20, 20' and transducer housing 22, 22' are formed preferably prevent luminescable material 232 from being exposed to wavelengths of ambient light which may excite luminescable material 232 (i.e., the material or materials are opaque to such wavelengths of radiation). Additionally, the material or materials of airway adapter 20, 20' and transducer housing 22, 22' preferably prevent luminescence detector 258 from being exposed to the same wavelengths of ambient radiation that luminescable material 232 emits upon being excited and that are quenched, or reduced in intensity, to a degree that is representative of an amount of oxygen or another analyzed gas or vaporized material to which luminescable material 232 is exposed. One or both of airway adapter 20, 20' and transducer housing 22, 22' may also be equipped with light sealing elements or optical filters that further prevent luminescable material 232 and luminescence detector 258 from being exposed to undesirable wavelengths of ambient radiation.

It is also preferred that the material or materials from which airway adapter 20, 20' and transducer housing 22, 22' are formed do not emit or fluoresce wavelengths of radiation that would either excite luminescable material 232 or be emitted therefrom upon exposure of airway adapter 20, 22' or transducer housing 22, 22' to either ambient radiation or to wavelengths of radiation that are emitted by infrared emitter 252, radiation source 256, or excited luminescable material 232.

Portions of airway adapter 20, 20' or transducer housing 22, 22', such as window 40, through which one or more wavelengths of radiation are to be transmitted are preferably formed from materials that do not absorb a substantial amount of the one or more wavelengths of radiation that are to be transmitted therethrough. Stated another way, these portions of airway adapter 20, 20' or transducer housing 22, 22' should be relatively transparent to the wavelengths of radiation that are indicative of an amount of one or more particular substances in the respiration of a patient. By way of example only and not to limit the use of polypropylene in airway adapter 20, 20' or in transducer housing 22, 22', while polypropylene has a high transmissivity for wavelengths that are used to detect carbon dioxide levels, polypropylene may not have good transmissivity for wavelengths of radiation that may be used to detect levels of other substances.

As discussed above and illustrated in FIGS. 1-5, airway adapter 20 may include a respiratory flow monitoring device 30 within first tubular portion 24 (most clearly seen in FIGS. 4 and 5). Respiratory flow monitoring device 30 of airway adapter 20 may comprise any known, suitable type of respiratory flow monitor. An exemplary respiratory flow monitoring device 30 includes a diametrically oriented, longitudinally extending strut 44 of axial length L and height HI within a tubular housing 46 of airway adapter 20. Strut 44 has first and second end faces 50 and 52, and first and second side faces 54 and 56.

It is contemplated that the end faces 50 and 52 may be substantially perpendicular to axis A, as shown in FIG. 5, and chamfered and rounded, as shown, so long as the end face configuration is symmetrical when viewed from above. The major characteristic of end faces 50 and 52, aside from symmetry, is that they do not incline toward notches 58 and 60 or otherwise collect or direct flow through flow monitoring device 30 toward notches 58 and 60 and pressure ports 62 and 66. End faces 50 and 52 are preferably aerodynamically designed so as to minimize resistance to the gas flow.

As shown in FIG. 5, side faces 54 and 56 of strut 44 are flat, again the major requirement being one of symmetry between the sides of strut 44, as with end faces 50 and 52.

Strut 44 also provides a position for pressure ports 62 and 66 and conditions the velocity profile of the flowing gas. Strut 44 is offset from an inner wall 48 of tubular housing 46 and is secured, at both ends, to inner wall 48.

The cross-sectional area of the 44 transverse to a bore axis A should be minimized. The minimization of this dimension is, however, constrained by the diameters of pressure ports 62 and 66. Typically, the cross-sectional area of strut 44 may be about five percent (5%) of the cross-sectional bore area of tubular housing 46 at the location of strut 44.

It should be noted that the diameter of the bore through tubular housing 46, depicted in FIGS. 4-5, is different between first tubular portion 24 and second tubular portion 26. This configuration accommodates a male connecting tube element, shown in broken lines and designated as M on the left-hand side of first tubular portion 24 of airway adapter 20, and a female connecting tube element F on the right-hand side of second tubular portion 26 of airway adapter 20. Also, the internal bores of first and second tubular portions 24 and 26 may be tapered to facilitate the release of plastic injection molded parts from a formed airway adapter 20.

Strut 44 further includes notch structures comprising substantially symmetrical first and second notches 58 and 60, both of which are located substantially on axis A of tubular housing 46, notches 58 and 60 extending axially inwardly from first and second end faces 50 and 52, respectively, and laterally through first and second side faces 54 and 56, respectively. A first pressure port 62 of a first lumen 64 opens into first notch 58, and a second pressure port 66 of a second lumen 68 opens into second notch 60. First and second lumens 64 and 68 comprise passages internal to strut 44, which extend into and through first and second male stems 72 and 74, respectively, on an exterior surface of tubular housing 46.

Airway adapter 20 is preferably oriented with first and second male stems 72 and 74 directed upward, such that water condensation and mucus do not clog or otherwise impair pressure ports 62 and 66.

Both pressure ports 62 and 66 face substantially perpendicular to axis A of tubular housing 46, notches 58 and 60 extend axially inwardly to a depth D, at least past pressure ports 62 and 66, and may so extend a distance equal to the height H2 of notches 58 and 60, which, in turn, should be less than or equal to four-tenths (4/10) of the height HI of the strut 44.

Back walls 78 and 80 of notches 58 and 60, respectively, may be arcuate or radiused, as shown in FIG. 5, or otherwise symmetrically shaped, as with the end faces 50 and 52. Back walls 78 and 80 may also have substantially planar surfaces.

Floors 82 and 84 and ceilings 86 and 88 of notches 58 and 60, respectively, are preferably substantially planar, or flat, as shown in FIG. 4, or may be otherwise symmetrically shaped. Likewise, the transition edges or lines between end faces 50 and 52 and notches 58 and 60 are preferably radiused, although they may alternatively be chamfered or beveled.

Back walls 78 and 80 of notches 58 and 60, respectively, together with restrictions (ridges or lands) 90 comprise a flow obstruction 76 and/or perturbation to the gas flow through flow monitoring device 30, which generates the differential pressure signal measured at first and second pressure ports 62 and 66. The measured differential pressure signal is from either pressure loss or from vena contracta, the contraction of the velocity profile of flowing gases, which is caused by flow obstruction 76. The differential pressure generated from the vena contracta can be modeled by standard fluid mechanics equations such as Euler's or Bernoulli's equation. The differential pressure signal generated from vena contracta is considered "lossless", meaning that the pressure is restored as the velocity profile is returned to the incident velocity profile.

Respiratory flow, as measured by flow monitoring device 30, is proportional to the square root of the differential pressure, as measured at pressure ports 62 and 66.

Flow obstruction 76 may be varied in a number of ways to yield a different magnitude of measured differential pressure for a given flow rate. First, the cross-sectional area of restrictions (ridges or lands) 90 may be increased or decreased in the plane perpendicular to axis A. Also, the distance from the center of first pressure port 62 to back wall 78 of notch 58 and, likewise, the distance from the center of the second pressure port 66 to back wall 80 of notch 60, may be varied to change the flow response characteristics. The magnitude of the differential pressure signal for a given flow rate can be further increased by reducing the cross-sectional bore area by necking down the inner wall 48 of tubular housing 46.

The length and width of strut 44 may be altered, as desired, to change flow characteristics. These flow characteristics include flow conditioning, signal strength, and signal stability. Ideally, the incident velocity profile to flow obstruction 76 should be the same regardless of the velocity profile incident to airway adapter 20. Signal stability may be compromised when unstable, multidimensional vortex formations are generated by flow obstruction 76. Strut 44 with notch means provides flow conditioning that yields some immunity to inlet velocity profile and yields a stable differential pressure signal in response to the gas flow.

Flow monitoring device 30 may be selectively modified to adapt to the conditions under which flow monitoring device 30 is to operate. In particular, the modification of the cross-sectional flow area in the vicinity of strut 44 may be employed to adjust the dynamic range of the respiratory flow monitoring device 30, as may modifications to the configurations of end faces 50 and 52 and back walls 78 and 80 of notches 58 and 60, and to the lines of transition between notches 58 and 60 and end faces 50 and 52 and side faces 54 and 56. It is preferred to use laterally extending, transversely oriented center (strut 44) restrictions (ridges or lands) 90 and a gradual inner wall transition in the strut area axial length to add symmetry to the flow pattern, normalize the flow, provide immunity to moisture, and provide better repeatability of readings. The notch height H2 or the length of strut 44 may be increased or decreased to accommodate a wider range of inlet conditions, such as might result from employment of flow monitoring device 30 with a variety of endotracheal tubes.

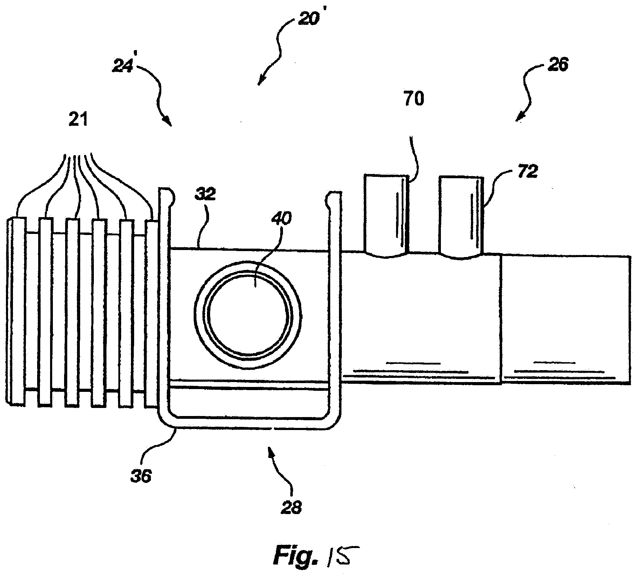

FIG. 15 illustrates a second embodiment of airway adapter 20' incorporating teachings of the present invention. Airway adapter 20' includes a plurality of ribs 92 around the outside diameter of a first portion 24' thereof. Ribs 92 preferably define a 22 mm diameter and reduce the weight of airway adapter 20' while providing uniform wall dimensions to facilitate injection molding of airway adapter 20'.

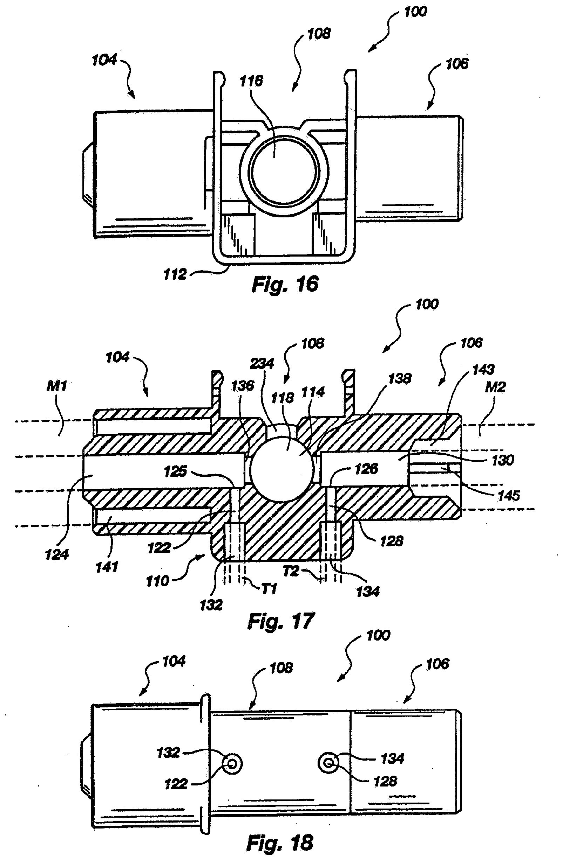

FIGS. 16-18 illustrate a third embodiment of an airway adapter 100 with reduced dead space relative to the embodiments disclosed previously herein. Airway adapter 100 is particularly suitable for use in situations where the respiratory tidal volume is extremely small, such as with newborn infants, although airway adapter 100 has equal utility in adult and pediatric respiratory monitoring.

As shown, airway adapter 100 is designed for connection between a patient ventilation device, such as an endotracheal tube inserted into a patient's trachea, attached to a first tubular portion 104 of airway adapter 100, and the tubing of a mechanical ventilator, attached at second tubular portion 106 of airway adapter 100. First and second tubular portions 104 and 106 have bores of varying diameter and of substantially circular cross-section. As shown in FIGS. 16-18, a gas concentration monitoring portion 108 of airway adapter 100 is disposed between first and second tubular portions 104 and 106.

Gas concentration monitoring portion 108 of airway adapter 100 provides a seat for a transducer housing (not shown), similar to transducer housing 22 shown in FIG. 1. An integral, U-shaped casing element 112 positively locates the transducer housing into position on airway adapter 100. In a preferred embodiment, the transducer housing snaps into place on airway adapter 100 without the need for tools to assemble or disassemble the transducer and airway adapter 100.

As illustrated, airway adapter 100 includes an annular recess 141 formed in first tubular portion 104. Annular recess 141 accommodates a male connecting tube element, shown in broken lines and designated as M1, on the left-hand side of first portion 104 of airway adapter 100. Second tubular portion 106 similarly includes a receptacle 143 configured to accommodate a second male connecting tube element M2, as shown in broken lines, which snaps into receptacle 143 by engaging a stepped slot 145 thereof. Elements M1 and M2 each include a bore of like diameter to the corresponding tubular chambers 130 and 124 of airway adapter 100. Elements M1 and M2 facilitate communication between airway adapter 100 and the airway of an individual and, if necessary, a respirator or other ventilation device.

Gas concentration monitoring portion 108 includes a luminescent sensing window 234 formed through U-shaped casing element 112. Window 234 facilitates the emission of excitation radiation from a source of excitation radiation within a transducer housing assembled with airway adapter 100, into airway adapter 100, and toward luminescable material (e.g., luminescable material 232 shown in FIG. 4) within airway adapter 100. In addition, window 234 facilitates the detection of luminescence emitted from the luminescable material of airway adapter 100 by a detector within the transducer housing, as discussed previously herein with reference to FIG. 6.

Gas concentration monitoring portion 108 also includes a first axially aligned window 116 and a second axially aligned window 118 (shown in FIG. 17 only) to allow an infrared radiation beam to travel from an infrared radiation emitter (See FIG.1) in the transducer housing transversely through a sampling chamber 114 in airway adapter 100 for monitoring gases, such as CO₂, N₂O, and anesthetic agents, as discussed previously herein.

Airway adapter 100 includes a respiratory flow monitoring device 110, which partially resides in first tubular portion 104, partially resides in second tubular portion 106, and partially resides in gas concentration monitoring portion 108.

Respiratory flow monitoring device 110, which is most clearly depicted in FIG. 17, also includes a first pressure port 125 of a first lumen 122, which opens into a first tubular chamber 124 of the tubular portion 104, and a second pressure port 126 of a second lumen 128 which opens into second tubular chamber 130. Lumens 122 and 128 extend to respective first and second recesses 132, 134, which are configured to minimize dead space and accommodate connecting tubes, shown in broken lines and designated as T1 and T2. Tubes T1 and T2 are connected to a flow monitor (not shown), which determines flow rate through a pressure differential detected between pressure ports 125 and 126. This pressure differential is produced through the use of necked-down ports 136 and 138 at the longitudinal ends of gas sampling chamber 114.

The heat generated by the radiation sources 252, 256 of transducer housing 22 (FIGS. 1 and 6) or from one or more other sources, which may be placed over airway adapter 100, should help to reduce the tendency of breath moisture to condense in airway adapter 100. The effects of water condensation are of particular concern in this embodiment due to its small volume and intended neonatal use; therefore, the airway adapter 100 should be positioned such that recesses 132 and 134 are directed upward to prevent clogging.

It has been found that this embodiment has many advantages, such as minimization of dead space and moldability in one piece.

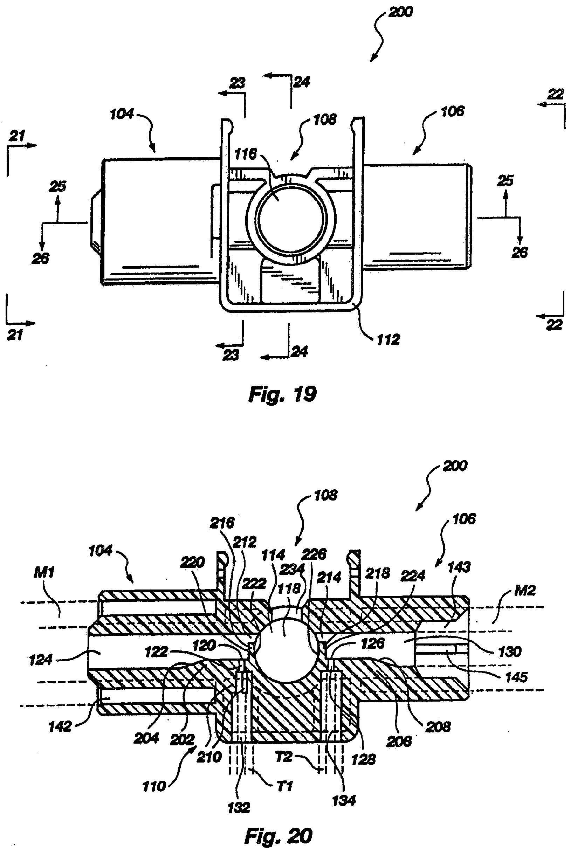

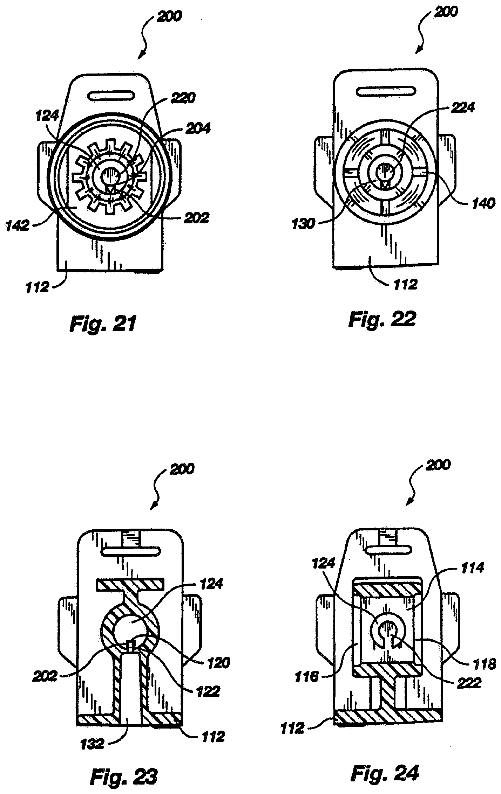

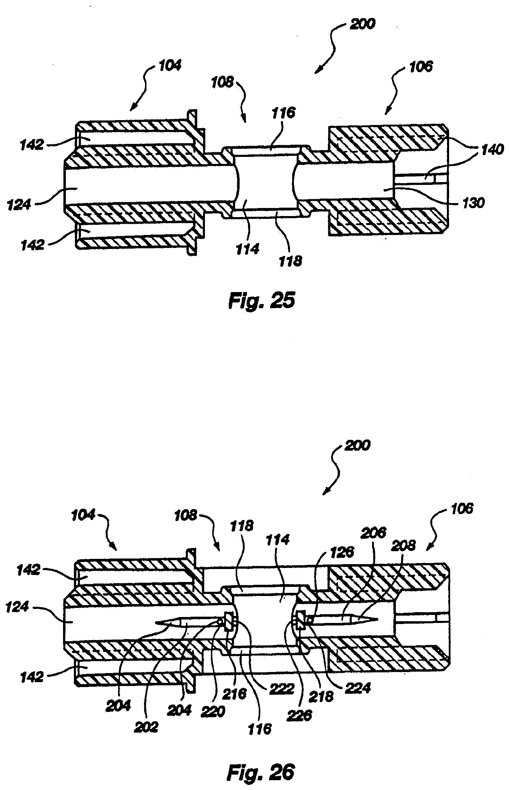

FIGS. 19-26 illustrate a fourth preferred embodiment of an airway adapter 200, which is similar to the airway adapter 100 of FIGS. 16-18. Therefore, components common to airway adapters 100 and 200, depicted in FIGS. 16-18 and FIGS. 19-26, respectively, retain the same numeric designation. Airway adapter 200 is particularly suitable for use in situations where the respiratory tidal volumes are extremely small, such as with newborn infants, although it has equal utility in pediatric and adult respiratory monitoring.

Airway adapter 200 is designed for connection between a patient ventilation device, such as an endotracheal tube inserted in a patient's trachea, attached to the first tubular portion 104, and the tubing of a mechanical ventilator, attached to second tubular portion 106. First and second tubular portions 104 and 106 have bores of varying diameter and of substantially circular cross-section, with gas concentration monitoring portion 108 positioned therebetween.

Gas concentration monitoring portion 108 of airway adapter 200 provides a seat for a transducer housing (not shown), similar to transducer housing 22 shown in FIG. 1. An integral, U-shaped casing element 112 positively locates the transducer housing into position on airway adapter 200. Preferably, the transducer housing snaps into place on airway adapter 200 without the need for tools to assemble or disassemble airway adapter 200 and the transducer housing.

In this embodiment, as with the embodiment of FIGS. 16-18, an annular recess 142 is formed in first tubular portion 104 to accommodate a male connecting tube element, shown in broken lines and designated as M1, on the left-hand side of first tubular portion 104 of airway adapter 200. Second tubular portion 106 includes a receptacle 143 that accommodates a second male connecting tube element M2, as shown in broken lines, which snaps into receptacle 143 by engaging a stepped slot 145 thereof. Elements M1 and M2 include bores of like diameter to bores of tubular chambers 124, 130. Elements M1 and M2 facilitate communication between airway adapter 200 and the airway of an individual and, if necessary, a respirator or other ventilation device.

Gas concentration monitoring portion 108 includes a luminescent sensing window 234 formed through U-shaped casing element 112. Window 234 facilitates the emission of excitation radiation from a source of excitation radiation within a transducer housing assembled with airway adapter 200, into airway adapter 200 toward luminescable material (e.g., luminescable material 232 shown in FIG. 4) within airway adapter 200. In addition, window 234 facilitates the detection of luminescence emitted from the luminescable material of airway adapter 200 by luminescence detector 258 within transducer housing 22, as discussed previously herein with reference to FIG. 6.

Gas concentration monitoring portion 108 also includes a first axially aligned window 116 and a second axially aligned window 118 to facilitate the transmittance of an infrared radiation beam from an infrared radiation emitter in the transducer housing, transversely through sampling chamber 114 in airway adapter 200 so that amounts of gases, such as CO₂, N₂O, and anesthetic agents in the respiration of an individual may be monitored as discussed previously herein.

Airway adapter 200 includes a respiratory flow monitoring device 110, which partially resides in first tubular portion 104, partially resides in second tubular portion 106, and partially resides in gas concentration monitoring portion 108. Respiratory flow monitoring device 110 includes a first pressure port 120 of a first lumen 122 that extends through a first strut 202 and opens into a first tubular chamber 124 of first tubular portion 104. First strut 202 has a tapered portion 204 directed toward first tubular portion 104 to minimize potential flow disturbances. Respiratory flow monitoring device 110 also includes a second pressure port 126 of a second lumen 128 that extends through a second strut 206 and opens into second tubular chamber 130. Second strut 206 has a tapered portion 208 directed toward second tubular portion 106 to minimize potential flow disturbances. Lumens 122 and 128 extend respectively to first and second recesses 132, 134.

Recesses 132 and 134 are configured to minimize dead space and to accommodate male connecting tubes, shown in broken lines and designated as T1 and T2. Recesses 132 and 134 may have internal ribs 210 to securely grip tubes T1 and T2. Tubes T1 and T2 are connected to a flow monitor (not shown), which determines flow rate through a pressure differential detected between pressure ports 120 and 126. This pressure differential is produced through the use of a first annular port 212 and a second annular port 214 at the longitudinal ends of gas sampling chamber 114. First annular port 212 is formed by a first restriction member 216 extending from first strut 202 and blocking a portion of first tubular chamber 124 of first tubular portion 104. The face surfaces 220, 222 of first restriction member 216 are preferably substantially perpendicular to the flow of the respiratory gas through airway adapter 200. Second annular port 214 is formed by a second restriction member 218 extending from second strut 206 and blocking a portion of second tubular chamber 130 of second tubular portion 106. Face surfaces 224, 226 of second restriction member 218 are preferably substantially perpendicular to the flow of the respiratory gas through the airway adapter 200. First restriction member 216 and second restriction member 218 can be any shape, such a circular, oval, rectangular, or the like. However, the preferred shape is a planar disk.

The heat generated by the radiation sources 252, 256 of transducer housing 22 (FIGS. 1 and 6) or from one or more other sources, which may be placed over airway adapter 200, should help to reduce the tendency of breath moisture to condense in airway adapter 200. The effects of water condensation are of particular concern in this embodiment due to its small volume and intended neonatal use; therefore, the airway adapter 200 should be positioned such that recesses 132 and 134 are directed upward to prevent clogging. It has been found that this embodiment has many advantages, such as minimization of dead space and moldability in one piece.

One of the uses of the multiple function airway adapter of the present invention is in a metabolic measurement system, which is a system that is capable of providing metabolic measurements, such as oxygen consumption or oxygen uptake, carbon dioxide production or carbon dioxide elimination, respiratory quotient (RQ), resting energy expenditure (REE), or any combination of such measurements. It should be noted that "oxygen update" and "oxygen consumption" are used synonymously, and are both represented by the expression "V̇_{O₂} " or, for simplicity "VO2". It should be noted that "carbon dioxide production" and "carbon dioxide elimination" are used synonymously, and both represented by the expression " V̇_{CO₂} "or for simplicity "VCO2."

Oxygen consumption is a measure of the amount of oxygen that the body uses in a given period of time, such as one minute. It is typically expressed as milliliters of oxygen used per minute (ml/min) or as milliliters of oxygen used per kilogram of body weight per minute (ml/kg/min). Measuring the rate of oxygen consumption is valuable, for example, in anesthesia and intensive care situations because it provides an indication of the sufficiency of a patient's cardiac and pulmonary function. VO₂ can also be used to monitor the fitness of an individual or athlete.

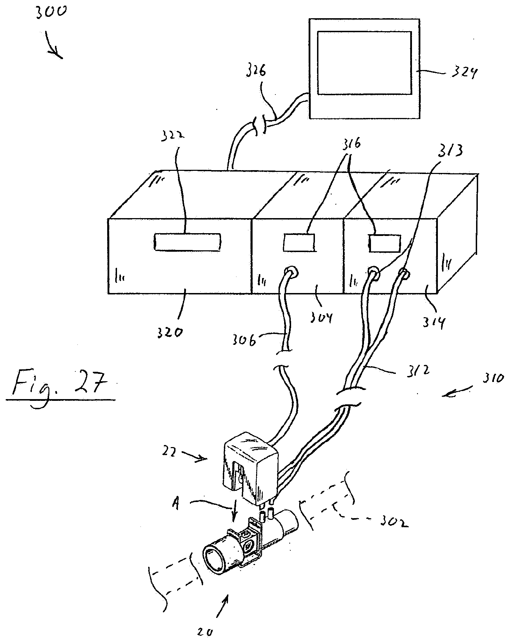

FIGS. 27-30 schematically illustrate various embodiments for a metabolic measurement system, generally indicated at 300, according to the principles of the present invention. Referring now to FIG. 27, metabolic measurement system 300 includes an airway adapter 20 and a separate transducer housing 22, as described in detail above. In this embodiment, airway adapter 20 is adapted to be coupled in series with a mainstream gas flow, such as a flow of gas carried by patient circuit or breathing circuit 302. Airway adapter 20 includes a housing have a bore defined therethrough to carry the mainstream gas flow through the airway adapter. A window, such as window 40, is defined in the housing providing optical access to the gas flow through the airway adapter. In a further embodiment, a pair of windows are provided, each window being provided on an opposite side of the airway adapter. An opening or window, such as opening/window 234, is defined in the housing providing optical access to the gas flow through the airway adapter.

Sensor head 22 is removably attached to airway adapter 20 as indicated by arrow A. The sensor head, as in the previous embodiment and described above, includes an infrared sensing system adapted to transmit or receive infrared radiation through the window or pair of windows, and a luminescence quenching system. The luminescence quenching system, as also described above, transmits excitation radiation through an opening, receives emitted radiation from a luminescence material through the same opening or a different opening. Detectors associated with the infrared sensing system and the luminescence quenching system provide signals indicative of a concentration of a gas in the gas flow through the airway adapter. In the embodiment illustrated in FIG. 27, the signals from the detectors associated with the infrared sensing system and the luminescence quenching system are provided to a gas monitoring module 304 via a hardwired communication link 306. Thus, monitoring module 304 is physically separated from sensor head 22.

Gas monitoring module 304 includes one or more processors that monitor or determine the concentration of a gas based on the signals from the detectors in the infrared sensing system and the luminescence quenching system. For example, the amount of carbon dioxide can be determined based on the signal from the infrared sensing system, and the amount of oxygen can be determined in gas monitoring module 304 based on the output of the luminescence quenching system. The CO₂ and/or O₂ levels can be output, for example, as waveforms or a numerical values. Monitoring module also provides signals to the radiation emitters in the infrared sensing system and the luminescence quenching system.

While a hardwire communication link 306 is shown, the present invention contemplates providing a wireless communication link between the portions of the infrared sensing system and the luminescence quenching system located in sensor head 22 and the processing elements located in gas monitoring module 304. In which case, power for the infrared sensing system and the luminescence quenching system can be provided via a power source, such as a battery contained in the sensor head, or via a power cable.

In addition, the present invention contemplates locating the processing elements that act on the signals produced by the detectors to determine the gas concentrations directly in the sensor head. An example of such a system is disclosed in U.S. patent no. 6,954,702, and in U.S. patent applications nos. 11/165,670 (publication no. US-2006-0009707-A1) and 11/368,832 (publication no. US-2006-0145078-A1). Thus, the signals provided by the sensor head would be the processed signals indicative of the gas concentrations, rather than raw signals produced by the detectors in the gas concentration monitoring systems.

Metabolic measurement system 300 also includes a flow measurement system, generally indicated at 310, that measures the flow of gas through airway adapter 20. In the illustrated embodiment, the flow measurement system measures flow by monitoring a pressure differential that is created across a flow restrictor disposed in the airway adapter. A pair of tubes 312 communicate each side of the flow restrictor to a pressure sensor (not shown), which, in the illustrated embodiment, is located in a flow processing module 314. A pair of ports or terminals 313 are provided that coupled to tubes 312 to communicate the pressure one each side of the flow restrictor with the pressure sensor or sensors in the flow processing module. The flow processing module includes processing elements that enables the rate of flow, or any other related parameter, or waveform thereof, to be determined based on the pressure monitored by the pressure sensor or sensor located in that module. The present invention contemplates that an optional input/output element 316 is provided on gas monitoring module 304 and/or flow monitoring module 314.

The output(s) of gas monitoring module 304 and flow monitoring module 314 are provided to a metabolic parameter processing module 320. More specifically, a processor in the metabolic parameter processing module receive signals from the infrared sensing system, the luminescence quenching system, and the flow measurement system either directly or via the gas monitoring module and the flow monitoring module 314. The processing in the metabolic parameter processing module uses these outputs to determine a metabolic parameter associate with the patient being monitored, such as VO₂, VCO₂, RQ, REE, or any other metabolic parameters or combinations thereof. The metabolic parameters can be displayed on an input/output device 322 provided on the metabolic parameter processing module. However, the present invention also contemplated providing a separate monitor or display 324 on which the output of gas monitoring module 304, flow monitoring module 314, and/or metabolic parameter processing module 320 are shown or otherwise provided.

In the illustrated embodiment, a hardwire link 326 is shown between metabolic parameter processing module 320 and monitor 324. It is to be understood, however, that this link can also be wireless. Moreover, other links, in place of or in addition to link 326, can be provided between monitor 324 and gas monitoring module 304 and/or flow monitoring module 314.

In an exemplary embodiment of the present invention, gas monitoring module 304, flow monitoring module 314, and metabolic parameter processing module 320 are configured such that each module is capable of physically joining another module and in so joining, creating a communication and/or power link between joined modules. This type of modularity provides a very flexible system for the end user.

Suppose for example, that a user wants to monitor only the flow for that patient. In which case, airway adapter 20 can be provided and coupled to only the flow monitoring module. Monitor 324 can be coupled to the flow monitoring module to display the flow waveform. If the user then decided to monitor the patient's CO₂, the gas monitoring module 304 can be provided. It can be linked to the flow monitoring module, if desired, or left separate from the flow monitoring module. The output of the gas monitoring module can also be displayed on the monitor. Finally, if the user decides to also monitor the patient's VO₂, the metabolic parameter processing module is added. Again, the metabolic parameter processing module can be separated from or linked with the gas and/or flow monitoring modules. However, the outputs of the gas and flow monitoring module must be provided to the metabolic parameter processing module, because, in this embodiment, it does not contain the processing elements necessary for interpreting the signals from the detectors in the gas and flow monitoring systems.

The present invention also contemplates providing other input/output capabilities for gas monitoring module 304, flow monitoring module 314, and metabolic parameter processing module 320. For example, each or all of these modules can includes displays or other visual or audio indicators to provide information to a user. Input devices, such has keypads, touch screens, buttons, switches, knobs, etc. can be provided for entering information into each module. Also, one or more communication links or terminals and other functionality can be provided for communicating a module with a remote location, either via a hardwire or wirelessly.

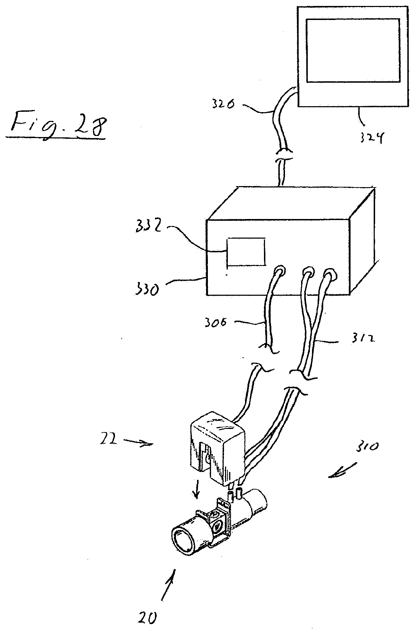

If less flexibility is desired, the functionality of the gas monitoring module 304, flow monitoring module 314, and metabolic parameter processing module 320 can be combined into a single housing, effectively combining these three modules. FIG. 28 illustrates an embodiment in which a single multi-parameter processing module 330 is provided that accomplishes the combined functions of the gas monitoring module, flow monitoring module, and metabolic parameter processing module. Monitor 324 can be built into module 330, as indicated by screen 332, or it can selectively coupled to this multi-purpose gas/flow monitoring system.

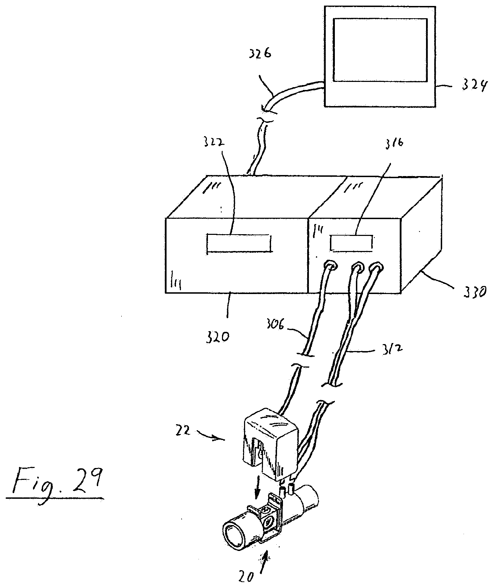

In the embodiment shown in FIG. 29, a combination gas/flow monitoring module 330 is provided. This module combines the functional aspects of gas monitoring module 304 and flow monitoring module 314. Gas/flow monitoring module 330 is selectively attachable to metabolic parameter processing module 320 to form a combined system.

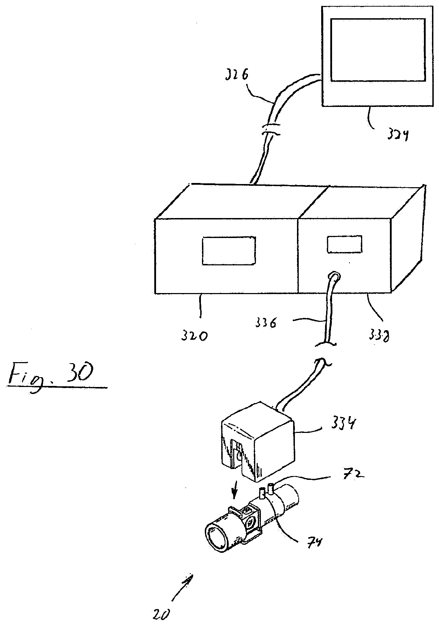

As shown, for example, in FIG. 30, the present invention further contemplates that the pressure sensing components of the flow monitoring system can be combined into a common sensor head 334. In which case, sensor head 334 includes pneumatic couplings (not shown) that couple to stems 72 and 74. As a result, each side of the flow restrictor in airway adapter 20 is in communication with the pressure sensor or sensors located in sensor head 334. An example, of a sensor head that includes pressure sensing components that can be mounted directly onto the airway adapter is disclosed in U.S. patent nos. 6,629,776 and 6,691,579.

In one embodiment, the processing element that communicates with the pressure sensor or sensors to determine the flow rate based on the output of the pressure sensor(s) is also provided in sensor head 334. In addition, a communication link 336 is provided to couple the output of the sensor head to a combined gas/flow sensing module 338. In an alternative embodiment, the processing element for producing the actual flow measurement based on the output of the pressure sensor(s) is located in gas/flow sensing module 338. The present invention also contemplates providing the processor or processors, which use the outputs of the IR sensing system and the luminescence quenching system and provide a meaningful or actual gas constituent measurements, in sensor head 334, gas/flow sensing module 338, or interspersed between these elements. It should be noted that if all of the signal processing is accomplished in sensor head 334, the final outputs can be provided directly to metabolic parameter processing module 320, thereby eliminating the need for gas/flow sensing module 338.

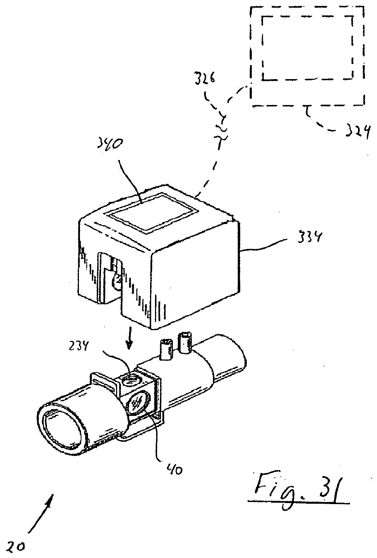

Carrying this concept one step further, the present invention also contemplates providing the metabolic parameter processing elements in sensor head 334. Thus, the entire metabolic measurement system need only include airway adapter 20 and sensor head 334, as shown, for example, in FIG. 31. Sensor head 334 includes the IR emitters detectors and process or the IR sensing system, the excitation radiation emitters and detectors in the luminescence quenching system, the flow detection elements of the flow measurement system, the processors and associated components required to use the output of these system to determine gas constituent and flow measurements and/or to determine whatever metabolic parameter is desired. An output device 340 can be provided directly on the sensor head, or a monitor 324 can be used to display the results of the analysis performed in the sensor head. Of course, the present invention contemplates providing other communication links between sensor head 334 and a remote device. Such communication links can be hardwired or wireless.

While a luminescence quenching system has been described, in detail, herein for sensing oxygen, the present invention also contemplates using other oxygen sensing systems in airway adapter 22, sensor head 22, 334, or both. For example, the known electro-chemical techniques (e.g. fuel cell) for sensing oxygen can be used in addition to or in place of the luminescence quenching system.

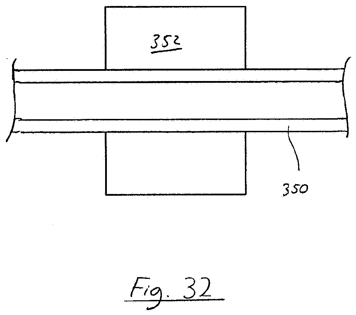

The present invention also contemplates using any form of flow sensing technique to detect the rate of flow of gas through the airway adapter, including those discussed in the Background of the Invention Section of the present invention. To emphasize this point, FIG. 32 is provided to schematically show a portion of a breathing circuit 350, which can be defined by an airway adapter, and a flow monitoring system 352 associated with this portion of the breathing circuit. Flow monitoring system 352 is any system capable of monitoring the flow of gas through the patient circuit. For example, flow monitoring system 352 can be an ultrasonic monitoring system that uses ultrasonic energy to determine the rate of flow of gas through breathing circuit 350. Flow monitoring system can also be a hot wire anemometer, that measure flow based on the cooling of a heated wire placed in the gas flow due to the gas passing around the wire. Of course, any combination flow measurement system can also be provided. In addition, the signal processing functions can b e provided in the flow monitoring system, i.e., at the flow sensor head proximate to the breathing circuit, or in a housing or module located remote from sensing portion the flow monitoring system.

A still further airway adapter that is capable of gas constituent and gas flow measurements suitable for use in the present invention is disclosed in U.S. provisional patent application no. 60/808,312, ("the '312 application") filed May 25, 2006. The airway adapter disclosed in the '312 application includes a housing having a flow restriction disposed in the flow path between first and second pressure ports. A pressure transducer in the form of an optical interferometer is associated with the pressure ports or the gas flow path between the pressure ports to provide the gas flow measurement. The gas constituent measurements are provided by a IR gas sensing system and/or a luminescence gas sensing system, or any other type of gas constituent sensing system disposed in the airway adapter.

By combining multiple different types of gas constituent and gas flow measurements in to a common housing, module, or sensing head, the present invention enables these measurements to be easily synchronized and used, in conjunction, to make metabolic measurements in real time. The modularity of the components provides flexibility in how the system is implemented and upgraded, while avoiding the need to have on hand more monitoring capability that is actually needed.

The airway adapter shown in FIGS. 27-31 for use in metabolic parameter monitoring system shown in these figures, corresponds to the airway adapter shown in FIGS. 1-14. It is to be understood that this airway adapter is only one example of airway adapters that are suitable for use in the metabolic parameter monitoring system of the present invention. For example, the airway adapter shown in FIGS. 17-26 is equally suitable for use in the metabolic parameter monitoring system embodiments shown in FIGS. 27-30.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A metabolic measurement system (300) comprising:
(a) an airway adapter (20, 100, 200, 334) adapted to be coupled in series with a mainstream gas flow comprising:
(1) a housing having a bore (34) defined therethrough to carry the mainstream gas flow through the airway adapter,
(2) a window (40) defined in the housing providing optical access to the gas flow through the airway adapter, and
(3) an opening (234) defined in the housing providing optical access to the gas flow through the airway adapter;
(b) a sensor head (22, 334) adapted to be removably attached to the airway adapter, the sensor head comprising:
(1) an infrared sensing system adapted to transmit or receive infrared radiation through the window, and
(2) a luminescence quenching system adapted to (i) transmit excitation radiation through the opening, (ii) receive emitted radiation from a luminescence material through the opening, or both (i) and (ii); and
(c) a flow measurement system (310, 352) adapted to be coupled in series with such a mainstream gas flow;
**characterized in that** the system further comprises:
(d) a gas monitoring module (304) adapted to receive a signal from the infrared sensing system and the luminescence quenching system, wherein the gas monitoring module includes a first processor adapted to determine a carbon dioxide waveform based on the signal from the infrared sensing system and an oxygen waveform based on the output of the luminescence quenching system;
(e) a flow processing module (314) adapted to receive a signal from the flow measurement system, wherein the flow processing module includes a second processor adapted to determine a rate, a volume, flow waveform, or any combination thereof for the mainstream gas flow; and
(f) a metabolic parameter processing module (320), wherein the metabolic parameter processing module includes a third processor adapted to receive signals from the infrared sensing system, the luminescence quenching system, and the flow measurement system, either directly or via the gas monitoring module and the flow monitoring module (314), wherein the third processor is adapted to determine a metabolic parameter including oxygen consumption (VO2), carbon dioxide production (VCO2), respiratory quotient (RQ), resting energy expenditure (REE), or any combination thereof; and
wherein the gas monitoring module (304), the flow monitoring module (314), and the metabolic parameter processing module (320) are configured such that each module is capable of physically joining another module and, in so joining, creating a communication and/or power link between the joined modules.

2. The system of claim 1, wherein at least a portion of the flow measurement system is disposed in the housing of the airway adapter, the sensor head, or both.

3. The system of claim 2, wherein the flow measurement system comprises:
a flow restrictor (76, 136, 138, 216, 218, 220, 222, 224) disposed in the housing and adapted to create a pressure drop across the flow restrictor; and
a pressure sensor adapted to measure a pressure associated with the pressure drop.

4. The system of claim 3, wherein the pressure sensor is disposed in the sensor head.

5. The system of claim 4, wherein the processor is spaced apart from the sensor head, and further comprising a hardwired or wireless communication link to communicate signals from the infrared sensing system, the luminescence quenching system, and the pressure sensor to the processor.

6. The system of claim 3, wherein the pressure sensor is disposed in the flow processing module (314), and wherein the system further comprises a pair of tubes (312) adapted to communicate each side of the flow restrictor (76, 136, 138, 216, 218, 220, 222, 224) to the pressure sensor.

7. The system of claim 6, wherein the flow processing module (314) comprises a pair of ports or terminals (313) adapted to be coupled to tubes (312).

8. The system of any of claims 1-6, wherein the gas monitoring module (304) and the flow monitoring module (314) are combined into a gas/flow monitoring module (330).

9. The system of any of claims 1-8, further comprising an output device (316, 322, 324, 332, 340) for providing the metabolic parameter in a human perceivable format, wherein the output device is coupled to the gas monitoring module, or the flow processing module.

10. The system of any of claims 1-8, further comprising an output device (316, 322, 324, 332, 340) for providing the metabolic parameter in a human perceivable format, wherein the output device is coupled to the processor of the metabolic parameter processing module.

11. The system of any of claims 1-9, wherein the luminescence quenching system is an oxygen sensing system associated with the opening and adapted to provide a signal indicative of a concentration of oxygen in the gas flow.

## Patentansprüche

1. Stoffwechselmesssystem (300), umfassend:
(a) einen Atemwegsadapter (20, 100, 200, 334), der ausgelegt ist, um in Reihe mit einer Hauptgasströmung gekoppelt zu werden, umfassend:
(1) ein Gehäuse mit einer Bohrung (34), die dadurch definiert ist, um die Hauptgasströmung durch den Atemwegsadapter zu führen,
(2) ein Fenster (40), das in dem Gehäuse, das einen optischen Zugang zur Gasströmung durch den Atemwegsadapter bereitstellt, definiert ist und
(3) eine Öffnung (234), die in dem Gehäuse, das einen optischen Zugang zur Gasströmung durch den Atemwegsadapter bereitstellt, definiert ist;
(b) einen Sensorkopf (22, 334), der dazu ausgelegt ist, um entfernbar an dem Atemwegsadapter angebracht zu werden, wobei der Sensorkopf umfasst:
(1) ein Infrarot-Erfassungssystem, das ausgelegt ist, um Infrarotstrahlung durch das Fenster zu senden oder zu empfangen, und
(2) ein Lumineszenz-Löschsystem, das ausgelegt ist, um (i) Anregungsstrahlung durch die Öffnung zu senden, (ii) emittierte Strahlung von einem Lumineszenzmaterial durch die Öffnung zu empfangen oder sowohl (i) als auch (ii); und
(c) ein Strömungsmesssystem (310, 352), das ausgelegt ist, um in Reihe mit einer solchen Hauptgasströmung gekoppelt zu werden;
**dadurch gekennzeichnet, dass** das System zudem umfasst:
(d) ein Gasüberwachungsmodul (304), das ausgelegt ist, um ein Signal von dem Infrarot-Erfassungssystem und dem Lumineszenz-Löschsystem zu empfangen, wobei das Gasüberwachungsmodul einen ersten Prozessor enthält, der ausgelegt ist, um eine Kohlendioxid-Wellenform basierend auf dem Signal von dem Infrarot-Erfassungssystem und eine Sauerstoffwellenform basierend auf der Ausgabe des Lumineszenz-Löschsystems zu bestimmen;
(e) ein Strömungsverarbeitungsmodul (314), das ausgelegt ist, um ein Signal von dem Strömungsmesssystem zu empfangen, wobei das Strömungsverarbeitungsmodul einen zweiten Prozessor enthält, der ausgelegt ist, um eine Rate, ein Volumen, eine Strömungswellenform oder eine beliebige Kombination davon für die Hauptgasströmung zu bestimmen; und
(f) ein Stoffwechselparameter-Verarbeitungsmodul (320), wobei das Stoffwechselparameter-Verarbeitungsmodul einen dritten Prozessor enthält, der ausgelegt ist, um Signale vom Infrarot-Erfassungssystem, dem Lumineszenz-Löschsystem und dem Strömungsmesssystem entweder direkt oder über das Gasüberwachungsmodul und das Strömungsüberwachungsmodul (314) zu empfangen, wobei der dritte Prozessor ausgelegt ist, um einen Stoffwechselparameter, einschließlich Sauerstoffverbrauch (VO2), Kohlendioxidproduktion (VCO2), Atmungsquotient (RQ), Ruheenergieaufwand (REE) oder einer beliebigen Kombination davon zu bestimmen; und
wobei das Gasüberwachungsmodul (304), das Strömungsüberwachungsmodul (314) und das Stoffwechselparameter-Verarbeitungsmodul (320) so konfiguriert sind, dass ein jedes Modul in der Lage ist, sich an ein anderes Modul physikalisch anzuschließen und durch das Anschließen eine Kommunikation und/oder Stromverbindung zwischen den angeschlossenen Modulen herzustellen.

2. System nach Anspruch 1, wobei mindestens ein Abschnitt des Strömungsmesssystems in dem Gehäuse des Atemwegsadapters, des Sensorkopfs oder beidem angeordnet ist.

3. System nach Anspruch 2, wobei das Strömungsmesssystem umfasst:
einen Strömungsbegrenzer (76, 136, 138, 216, 218, 220, 222, 224), der in dem Gehäuse angeordnet und ausgelegt ist, um einen Druckabfall über den Strömungsbegrenzer zu erzeugen; und
einen Drucksensor, der ausgelegt ist, um einen mit dem Druckabfall assoziierten Druck zu messen.

4. System nach Anspruch 3, wobei der Drucksensor in dem Sensorkopf angeordnet ist.

5. System nach Anspruch 4, wobei der Prozessor von dem Sensorkopf beabstandet ist und zudem umfassend eine festverdrahtete oder drahtlose Kommunikationsverbindung, um Signale von dem Infrarot-Erfassungssystem, dem Lumineszenz-Löschsystem und dem Drucksensor an den Prozessor zu kommunizieren.

6. System nach Anspruch 3, wobei der Drucksensor in dem Strömungsverarbeitungsmodul (314) angeordnet ist und wobei das System zudem ein Paar von Rohren (312) umfasst, das dazu ausgelegt ist, um eine jede Seite des Strömungsbegrenzers (76, 136, 138, 216, 218, 220, 222, 224) zum Drucksensor zu kommunizieren.

7. System nach Anspruch 6, wobei das Strömungsverarbeitungsmodul (314) ein Paar von Ports oder Anschlüssen (313) umfasst, die dazu ausgelegt sind, um mit Rohren (312) gekoppelt zu werden.

8. System nach einem der Ansprüche 1-6, wobei das Gasüberwachungsmodul (304) und das Strömungsüberwachungsmodul (314) zu einem Gas/Strömungsüberwachungsmodul (330) kombiniert sind.

9. System nach einem der Ansprüche 1-8, zudem umfassend eine Ausgabevorrichtung (316, 322, 324, 332, 340) zum Bereitstellen des Stoffwechselparameters in einem vom Menschen wahrnehmbaren Format, wobei die Ausgabevorrichtung mit dem Gasüberwachungsmodul, oder dem Strömungsverarbeitungsmodul gekoppelt ist.

10. System nach einem der Ansprüche 1-8, zudem umfassend eine Ausgabevorrichtung (316, 322, 324, 332, 340) zum Bereitstellen des Stoffwechselparameters in einem vom Menschen wahrnehmbaren Format, wobei die Ausgabevorrichtung mit dem Prozessor des Stoffwechselparameter-Verarbeitungsmoduls gekoppelt ist.

11. System nach einem der Ansprüche 1-9, wobei das Lumineszenz-Löschsystem ein Sauerstofferfassungssystem ist, das mit der Öffnung assoziiert ist und ausgelegt ist, um ein Signal bereitzustellen, das indikativ für eine Sauerstoffkonzentration in der Gasströmung ist.

## Revendications

1. Système de mesure métabolique (300) comprenant :
(a) un adaptateur pour voies respiratoires (20, 100, 200, 334), adapté pour être couplé en série à un flux gazeux principal, comprenant :
(1) un logement comportant un alésage (34) défini à travers celui-ci pour transporter le flux gazeux principal à travers l'adaptateur pour voies respiratoires,
(2) une fenêtre (40) définie dans le logement fournissant un accès optique au flux gazeux à travers l'adaptateur pour voies respiratoires, et
(3) une ouverture (234) définie dans le logement fournissant un accès optique au flux gazeux à travers l'adaptateur pour voies respiratoires ;
(b) une tête de capteur (22, 334) adaptée pour être fixée de manière amovible à l'adaptateur pour voies respiratoires, la tête de capteur comprenant :
(1) un système de détection infrarouge adapté pour transmettre ou recevoir un rayonnement infrarouge à travers la fenêtre, et
(2) un système d'extinction de la luminescence adapté pour (i) transmettre un rayonnement d'excitation à travers l'ouverture, (ii) recevoir le rayonnement émis à partir d'un matériau luminescent à travers l'ouverture, ou pour à la fois (i) et (ii) ; et
(c) un système de mesure du débit (310, 352) adapté pour être couplé en série au dit flux gazeux principal ;
**caractérisé en ce que** le système comprend de plus :
(d) un module de contrôle des gaz (304) adapté pour recevoir un signal provenant du système de détection infrarouge et du système d'extinction de la luminescence, dans lequel le module de contrôle des gaz inclut un premier processeur adapté pour déterminer une forme d'onde de dioxyde de carbone basée sur le signal provenant du système de détection infrarouge et une forme d'onde d'oxygène basée sur la sortie du système d'extinction de la luminescence ;
(e) un module de traitement du débit (314) adapté pour recevoir un signal provenant du système de mesure du débit, dans lequel le module de traitement du débit inclut un second processeur adapté pour déterminer un débit, un volume, une forme d'onde de débit, ou toute combinaison de ceux-ci pour le flux gazeux principal ; et
f) un module de traitement (320) des paramètres métaboliques, dans lequel le module de traitement des paramètres métaboliques inclut un troisième processeur adapté pour recevoir des signaux provenant du système de détection infrarouge, du système d'extinction de la luminescence et du système de mesure du débit, soit directement, soit par l'intermédiaire du module de contrôle des gaz et du module de contrôle du débit (314), dans lequel le troisième processeur est adapté pour déterminer un paramètre métabolique incluant la consommation d'oxygène (VO2), la production de dioxyde de carbone (VCO2), le quotient respiratoire (RQ), la dépense d'énergie au repos (REE), ou toute combinaison de ceux-ci ; et
dans lequel le module de contrôle des gaz (304), le module de contrôle du débit (314) et le module de traitement (320) des paramètres métaboliques sont configurés de telle sorte que chaque module est capable de se relier physiquement à un autre module et, en se reliant ainsi, de créer une communication et/ou une connexion électrique entre les modules reliés.

2. Système selon la revendication 1, dans lequel au moins une partie du système de mesure du débit est disposée dans le logement de l'adaptateur pour voies respiratoires, de la tête de capteur, ou des deux.

3. Système selon la revendication 2, dans lequel le système de mesure du débit comprend :
un réducteur de débit (76, 136, 138, 216, 218, 220, 222, 224) disposé dans le logement et adapté pour créer une chute de pression à travers le réducteur de débit ; et
un capteur de pression adapté pour mesurer une pression associée à la chute de pression.

4. Système selon la revendication 3, dans lequel le capteur de pression est disposé dans la tête du capteur.

5. Système selon la revendication 4, dans lequel le processeur est espacé de la tête du capteur, et comprenant de plus une liaison de communication câblée ou sans fil pour communiquer des signaux du système de détection infrarouge, du système d'extinction de la luminescence et du capteur de pression au processeur.

6. Système selon la revendication 3, dans lequel le capteur de pression est disposé dans le module de traitement du débit (314), et dans lequel le système comprend de plus une paire de tubes (312) adaptés pour communiquer chaque côté du réducteur de débit (76, 136, 138, 216, 218, 220, 222, 224) au capteur de pression.

7. Système selon la revendication 6, dans lequel le module de traitement du débit (314) comprend une paire de ports ou de bornes (313) adaptés pour être couplés aux tubes (312).

8. Système selon l'une quelconque des revendications 1-6, dans lequel le module de contrôle des gaz (304) et le module de contrôle du débit (314) sont combinés en un module de contrôle des gaz/du débit (330).

9. Système selon l'une quelconque des revendications 1-8, comprenant de plus un dispositif de sortie (316, 322, 324, 332, 340) servant à fournir le paramètre métabolique dans un format perceptible par l'homme, dans lequel le dispositif de sortie est couplé au module de contrôle des gaz ou au module de traitement du débit.

10. Système selon l'une quelconque des revendications 1-8, comprenant de plus un dispositif de sortie (316, 322, 324, 332, 340) servant à fournir le paramètre métabolique dans un format perceptible par l'homme, dans lequel le dispositif de sortie est couplé au processeur du module de traitement des paramètres métaboliques.

11. Système selon l'une quelconque des revendications 1-9, dans lequel le système d'extinction de la luminescence est un système de détection d'oxygène associé à l'ouverture et adapté pour fournir un signal indicatif d'une concentration d'oxygène dans le flux gazeux.
